# EUROPEAN PATENT APPLICATION

(11) **EP 4 775 228 A1**
(43) Date of publication of application: **15.07.2026**
(21) Application number: 25382006.2
(22) Date of filing: 08.01.2025
(51) Int. Cl.: A61K 48/00, A61P 9/06, C07K 14/47, C12N 15/86

(54) **METHODS FOR IMPROVING CARDIAC FUNCTION IN ARRHYTHMOGENIC RIGHT VENTRICULAR CARDIOMYOPATHY TYPE 5**

(71) Applicant: Centro Nacional de Investigaciones Cardiovasculares Carlos III (F.S.P.), 28029 Madrid (ES); Fundación para la Investigación Biomédica del Hospital Universitario Puerta de Hierro Majadahonda, 28222 Majadahonda (Madrid) (ES); University of Florida Research Foundation, Incorporated, Gainesville, FL 32601 (US)
(72) Inventor: Byrne, Barry John, Gainesville, 32607 (US); Corti, Manuela, Gainesville, 32615 (US); Lara Pezzi, Enrique, 28029 Madrid (ES); Lalaguna Díaz, Laura, 28029 Madrid (ES); García Pavía, Pablo, 28222 Majadahonda (ES)
(74) Representative: Pons IP

(57) **Abstract**

Arrhythmogenic right ventricular cardiomyopathy type 5 (ARVC5) is the most aggressive type of ARVC, caused by a fully penetrant missense mutation (p.S358L) in TMEM43. Pathologically, the disease is characterized by dilation of the cardiac chambers and fibro-fatty replacement of the myocardium, which results in heart failure and sudden cardiac death. Current therapeutic options are limited and no specific therapies targeting the primary cause of the disease have been proposed. Overexpression of WT-TMEM43 and a codon-optimized self-complementary adeno-associated virus (AAV) bearing WT-TMEM43 improve the pathological phenotype of ARVC5, offering a promising and specific therapy for patients suffering from this highly lethal disease.

## Description

### TECHNICAL FIELD

The present invention belongs to the field of medicine. More specifically, to the treatment of cardiac diseases and, more specifically, to improve the cardiac function in arrhythmogenic right ventricular cardiomyopathy type 5.

### BACKGROUND OF THE INVENTION

Arrhythmogenic right ventricular cardiomyopathy (ARVC) is an inherited cardiac disorder characterized by chamber dilation and fibrofatty replacement of the myocardium. Individuals present with dizziness, ventricular arrhythmias and sudden cardiac death (SCD) (Corrado D et al. Arrhythmogenic Cardiomyopathy. Circ Res 121, 785-802 (2017)). ARVC-associated genetic variants mainly affect proteins of the cardiac desmosome, triggering aberrant signaling pathways that elicit fibrosis and inflammation (Arbelo E. et al. 2023 ESC Guidelines for the management of cardiomyopathies: Developed by the task force on the management of cardiomyopathies of the European Society of Cardiology (ESC). Eur Heart J 44, 3503-3626 (2023), Marcus FI et al. Diagnosis of arrhythmogenic right ventricular cardiomyopathy/dysplasia: Proposed Modification of the Task Force Criteria. Eur Heart J 31, 806-814 (2010)).

Among the different causes of ARVC, genetic variants of transmembrane protein 43 (TMEM43) are responsible for the most aggressive subtype of the disease, commonly referred to as ARVC type 5 or ARVC5 (James CA et al. International Evidence Based Reappraisal of Genes Associated with Arrhythmogenic Right Ventricular Cardiomyopathy Using the Clinical Genome Resource Framework. Circ Genom Precis Med 14, E003273 (2021); Merner ND et al. Arrhythmogenic right ventricular cardiomyopathy type 5 is a fully penetrant, lethal arrhythmic disorder caused by a missense mutation in the TMEM43 gene. Am J Hum Genet 82, 809-821 (2008)). This highly lethal disease has a very poor prognosis, especially in men who show a median survival of only 42 years (Hodgkinson, K. et al. The natural history of a genetic subtype of arrhythmogenic right ventricular cardiomyopathy caused by a p.S358L mutation in TMEM43. Clin Genet 83, 321-331 (2013); Hodgkinson KA et al. Long-Term Clinical Outcome of Arrhythmogenic Right Ventricular Cardiomyopathy in Individuals With a p.S358L Mutation in TMEM43 Following Implantable Cardioverter Defibrillator Therapy. Circ Arrhythm Electrophysiol 9, e003589 (2016). The affected subjects present an autosomal dominant, fully penetrant form of ARVC, with common left ventricle (LV) involvement (Hodgkinson K *et al.,* 2013; Hodkinson KA *et al.,* 2013; Dominguez F et al. Clinical characteristics and determinants of the phenotype in TMEM43 arrhythmogenic right ventricular cardiomyopathy type 5. Heart Rhythm 17, 945-954 (2020)).

ARVC5 is caused by mutations in TMEM43 (Merner ND et al. Arrhythmogenic right ventricular cardiomyopathy type 5 is a fully penetrant, lethal arrhythmic disorder caused by a missense mutation in the TMEM43 gene. Am J Hum Genet 82, 809-821 (2008)). The first and most common mutation causing ARVC5 (p.S358L) was initially identified in 2008 in Newfoundland (Canada), but subsequent cases have been diagnosed around the globe, including Spain, UK, Germany and Denmark (Merner ND *et al.,* 2008; Haywood AFM et al. Recurrent missense mutations in TMEM43 (ARVD5) due to founder effects cause arrhythmogenic cardiomyopathies in the UK and Canada. Eur Heart J 34, 1002-1011 (2013); Dominguez F. et al. Clinical characteristics and determinants of the phenotype in TMEM43 arrhythmogenic right ventricular cardiomyopathy type 5. Heart Rhythm 17, 945-954 (2020); Milting H. et al. The TMEM43 Newfoundland mutation p.S358L causing ARVC-5 was imported from Europe and increases the stiffness of the cell nucleus. Eur Heart J 36, 872-881 (2015). TMEM43 has 4 transmembrane domains and the affected serine in ARVC5 (Ser358) lies within the third of these domains. In a previous work, it was reported a transgenic mouse model of ARVC5 that recapitulates the main characteristics of the human disease (Padrón-Barthe L. et al. Severe Cardiac Dysfunction and Death Caused by Arrhythmogenic Right Ventricular Cardiomyopathy Type 5 Are Improved by Inhibition of Glycogen Synthase Kinase-3β. Circulation 140, 1188-1204 (2019)). Currently, there are no targeted therapies for ARVC5 and only ICD implantation is offered as a prophylactic treatment. If patients survive ventricular arrhythmia and/or SCD, they often develop end-stage heart failure, and their only alternative is heart transplantation (Arbelo E *et al.,* 2023; Marcus Fl *et al.,* 2010).

It is therefore a need to develop a treatment for ARVC5.

### DESCRIPTION OF THE INVENTION

### BRIEF DESCRIPTION OF THE INVENTION

The present invention shows the efficacy of wild type (WT) TMEM43 overexpression as a therapy for the treatment of ARVC5. It was observed that overexpression of WT-TMEM43 delays the onset of ARVC5 phenotype in a transgenic mouse model, significantly improving cardiac contraction, reducing electrocardiographic (ECG) abnormalities and fibrosis, and increasing lifespan. As a first step towards translation of this finding to the clinics, an adeno-associated virus (AAV) expressing a codon-optimized WT-TMEM43 was generated. This construct effectively improves both functional and ECG abnormalities caused by S358L-TMEM43.

In a first aspect, the invention is directed to a nucleic acid comprising the human TMEM43 coding sequence as set forth in SEQ ID NO: 5

In a second aspect, the invention is directed to an adeno-associated virus (AAV) vector comprising the human TMEM43 coding sequence as set forth in SEQ ID NO: 5

In a third aspect, the invention is directed to a pharmaceutical composition comprising the nucleic acid of the first aspect of the invention or the AAV vector comprising the human TMEM43 coding sequence as set forth in SEQ ID NO: 5.

In a fourth aspect, the invention is directed to the pharmaceutical compositions comprising the human TMEM43 coding sequence as set forth in SEQ ID NO: 5 for use as a medicament.

In a fifth aspect, the invention is directed to the pharmaceutical compositions comprising the human TMEM43 coding sequence as set forth in SEQ ID NO: 5 for use in a method of treating an arrhythmogenic cardiomyopathy.

A sixth aspect of the invention is directed to a method of increasing the expression of human TMEM43 of SEQ ID NO: 2 in a target cell, comprising contacting a target cell with a plurality of AAV particles comprising a nucleic acid expression cassette comprising a functional human TMEM43 coding sequence as set forth in SEQ ID NO: 1.

A seventh aspect of the invention is directed to a nucleic acid overexpression cassette comprising a functional human TMEM43 coding sequence as set forth in SEQ ID NO: 1, for use in a method for treating a cardiac disease.

### BRIEF DESCRIPTION OF THE FIGURES

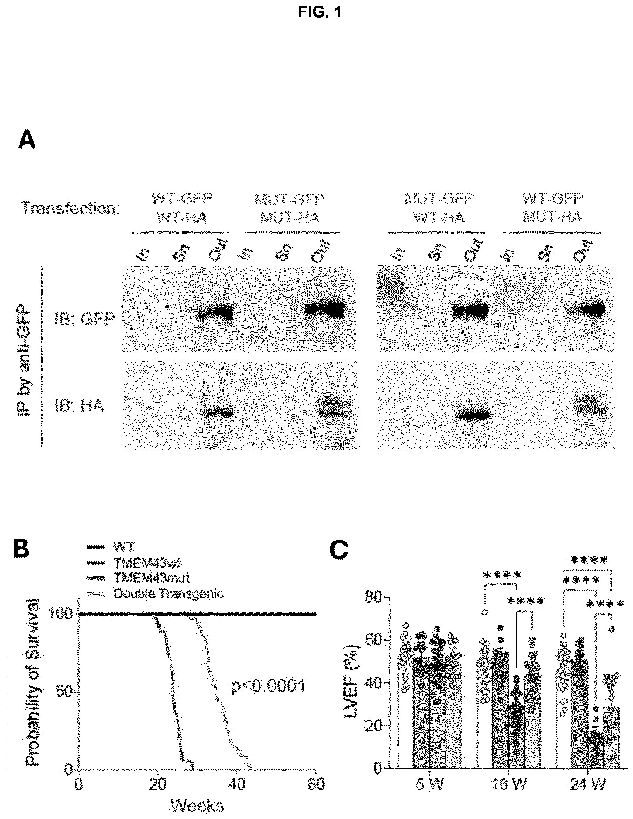

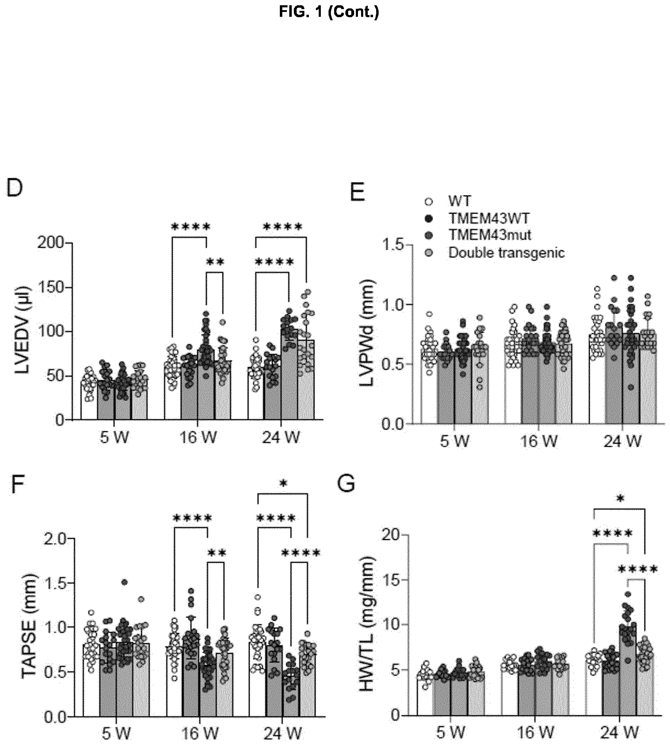

**Figure 1****. Wild type TMEM43 overexpression improves systolic function and lifespan in ARVC5 mice.** (A) Anti-GFP co-immunoprecipitation in protein extracts from P19 cells transfected with GFP- and HA-tagged wild type (WT, blue) and/or S358L (MUT, red) TMEM43. Input (In), supernatant (Sn) and output (Out) from the IP were analyzed by western blot. TMEM43 was detected by western blot with antibodies against GFP and HA. IP, immunoprecipitation; IB, immunoblot. (B) Survival rate of wild type mice (WT, n=22), mice overexpressing either WT-TMEM43 (TMEM43wt, n=20) or S358L-TMEM43 (TMEM43mut, n=35) and double transgenic mice (Double transgenic, n=35). The indicated P value was obtained with a log-rank test. (C-F) Left ventricular ejection fraction (LVEF, C), end-diastolic volume (LVEDV, D), left ventricle posterior wall thickness in diastole (LVPWd, E) and tricuspid annular plane systolic excursion (TAPSE, F) measured by echocardiography at 5, 16 and 24 weeks of age. (G) Ratio of heart weight to tibial length (HW/TL) determined at 5, 16 and 24 weeks. Data are shown as mean ± SD. *p<0.05; **p<0.01; ***p<0.001; ****p<0.0001. Two-way ANOVA was used, followed by Bonferroni post-test (C-G). Since there is no significant difference between WT and TMEM43wt mice, statistical comparisons between TMEM43wt and TMEM43mut or double transgenic mice are omitted to enhance clarity of the plots.

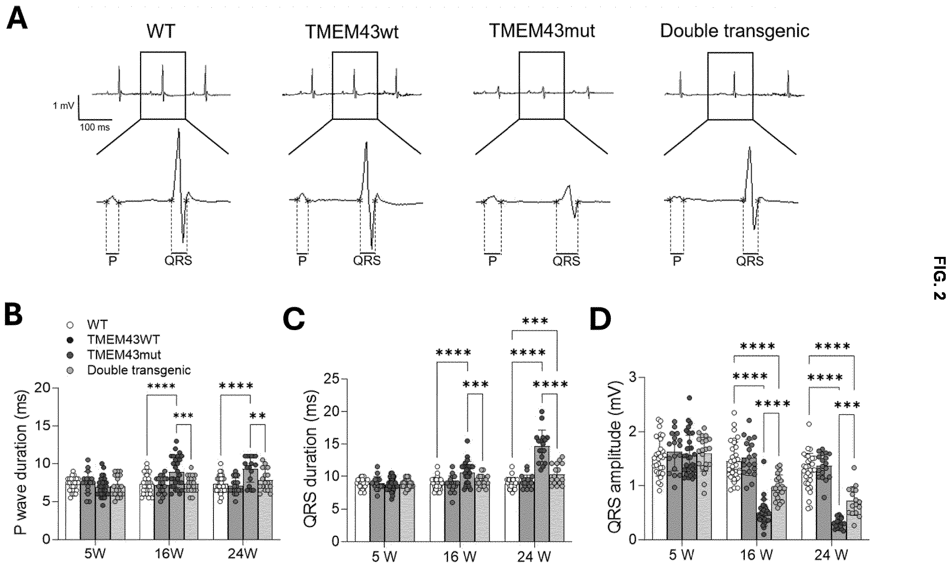

**Figure 2****. Wild type TMEM43 overexpression reduces ECG abnormalities in ARVC5 mice.** (A) Surface ECG traces and magnifications from lead II in 16 weeks old mice. (B, C, D) P wave duration (B) and QRS duration and amplitude (C and D) were measured from lead II surface electrocardiograms. Data are shown as mean ± SD. *p<0.05; **p<0.01; ***p<0.001; ****p<0.0001. Two-way ANOVA was used, followed by Bonferroni post-test. Since there is no significant difference between WT and TMEM43wt mice, statistical comparisons between TMEM43wt and TMEM43mut or double transgenic mice are omitted to enhance clarity of the plots.

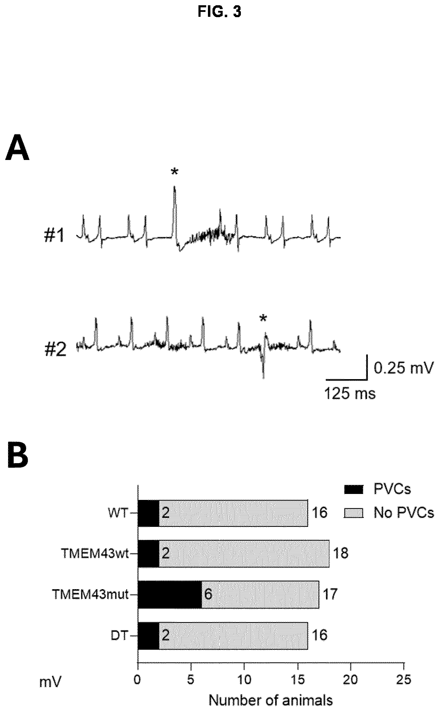

**Figure 3****. Arrhythmic events in transgenic mice.** (A) Lead II ECG traces from two TMEM43mut mice at 16 weeks showing premature ventricular contractions (PVCs, asterisks). (B) Number of animals showing PVCs at 5-, 16- or 24-weeks during follow-up.

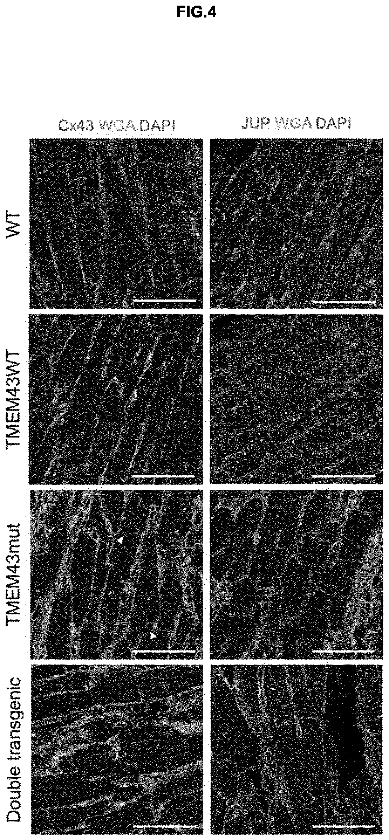

**Figure 4****. Wild type TMEM43 overexpression partially prevents Cx43 mislocalization.** Immunofluorescence analysis of connexin 43 (Cx43, left) or plakoglobin (JUP, right) and wheat germ agglutinin (WGA) in 16 weeks old WT, TMEM43wt, TMEM43mut and double transgenic mice. Scale bar, 50 µm. Arrowhead indicates mislocalised Cx43.

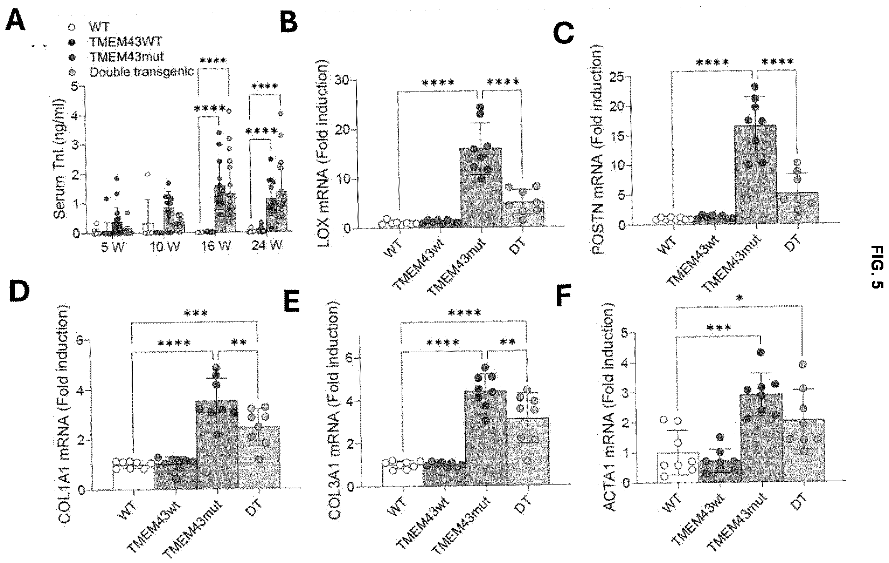

**Figure 5****. Wild type TMEM43 overexpression results in reduced cardiomyocyte death and downregulation of fibrosis-related genes.** (A) Serum troponin I (Tnl) levels were measured by ELISA in mice at 5, 10, 16 and 24 weeks of age. (B-F) mRNA expression levels of LOX (B), POSTN (C), COL1A1 (D), COL3A1 (E) and ACTA1 (F) was determined by qRT-PCR in 16 weeks old mouse hearts. Data are shown as mean ± SD. *p<0.05; **p<0.01; ***p<0.001; ****p<0.0001. Two-way ANOVA (A) or one-way ANOVA (B-F) were used, followed by Bonferroni post-test. Since there is no significant difference between WT and TMEM43wt mice, statistical comparisons between TMEM43wt and TMEM43mut or double transgenic mice are omitted to enhance clarity of the plots.

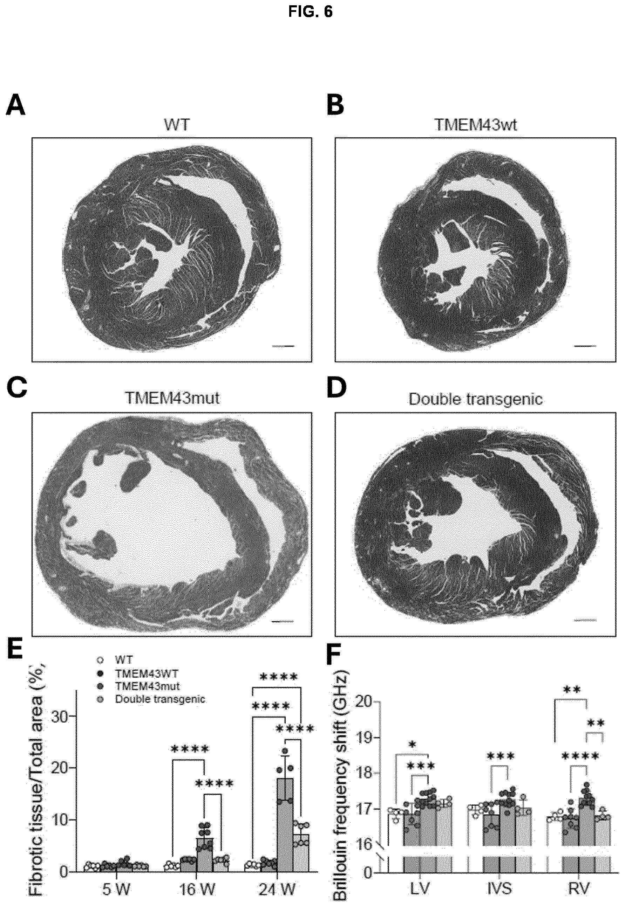

**Figure 6****. Wild type TMEM43 overexpression reduces myocardial fibrosis in ARVC5 mice.** (A-D) Collagen content in myocardial cross-sections from WT (A), TMEM43wt (B), TMEM43mut (C) and double transgenic (D) mice was analyzed at 24 weeks of age by Masson trichrome staining; bar 500 µm. (E) Quantification of fibrotic tissue from Masson trichrome staining. (F) Brillouin frequency shift was obtained from myocardial sections of 24 weeks old mice. LV, left ventricle; IVS, interventricular septum; RV, right ventricle. Data are shown as mean ± SD. *p<0.05; **p<0.01; ***p<0.001; ****p<0.0001. Two-way ANOVA was used, followed by Bonferroni post-test. Since there is no significant difference between WT and TMEM43wt mice, statistical comparisons between TMEM43wt and TMEM43mut or double transgenic mice are omitted to enhance clarity of the plots.

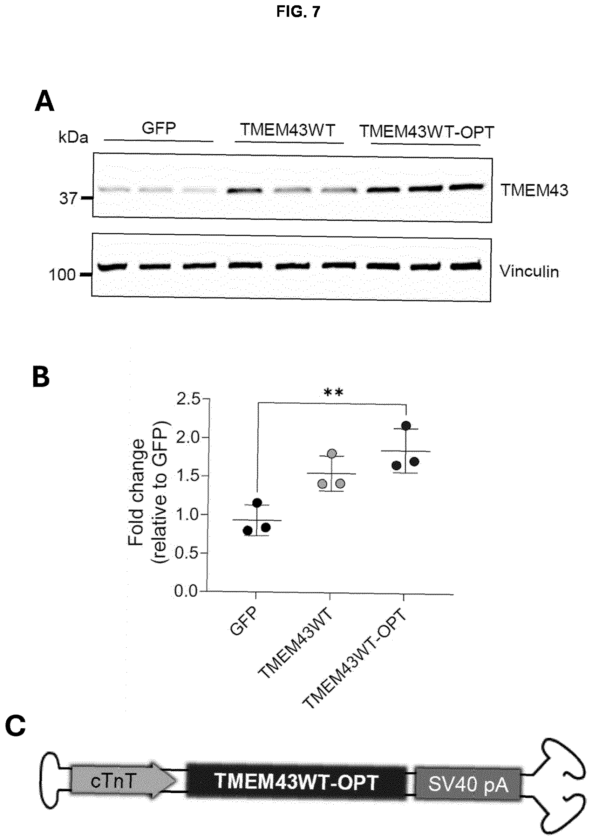

**Figure 7****. Codon-optimization of WT-TMEM43 increases protein expression.** (A-B) Western blot analysis (A) and TMEM43 quantification (B) of lysates from HL1 cells transfected with human TMEM43 (TMEM43WT), codon optimized TMEM43 (TMEM43WT-OPT) or control (GFP) plasmids. All of them carried chicken cardiac troponin T (cTnT) as a promoter. (C) Illustration of the self-complementary AAV vector used to deliver optimized human WT-TMEM43 in mice. Data are shown as mean ± SD. *p<0.05; **p<0.01; ***p<0.001; ****p<0.0001. One-way ANOVA was used, followed by Bonferroni post-test.

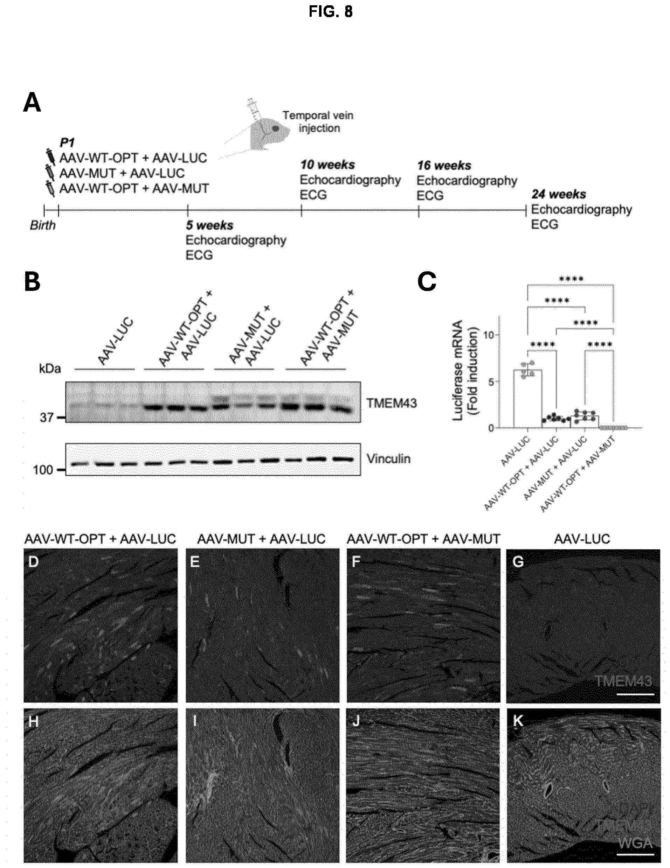

**Figure 8****. Delivery of codon-optimized WT-TMEM43 using an adeno-associated viral vector (AAV) shows high expression in the myocardium.** (A) Experimental design of self-complementary AAV administration and outcome analyses. A total viral load of 5e10 viral particles (VP) was administered into postnatal day 1 (P1) mice through the temporal vein as follows: AAV carrying TMEM43WT-OPT (AAV-WT-OPT) + AAV carrying luciferase (AAV-LUC) (2.5e10 VP each), AAV carrying S358L-TMEM43 (AAV-MUT) + AAV-LUC (2.5e10 VP each) or AAV-WT-OPT + AAV-MUT (2.5e10 VP each). (B, C) TMEM43 expression was analyzed by western blot (B) and luciferase mRNA expression levels were determined by qRT-PCR (C) in 16 weeks old mice hearts after i.v. delivery of AAV-LUC (5e10 VP/neonate), AAV-WT-OPT + AAV-LUC (2.5e10 VP/neonate each), AAV-MUT + AAV-LUC (2.5e10 VP/neonate each) or AAV-WT-OPT + AAV-LUC (2.5e10 VP/neonate each). (D-G) TMEM43 immunofluorescence analysis and WGA co-stains in myocardial sections of 16 weeks old mice after neonatal injection of AAVs; bar, 200 µm. Data are shown as mean ± SD (C). *p<0.05; **p<0.01; ***p<0.001; ****p<0.0001. One-way ANOVA was used, followed by Bonferroni post-test.

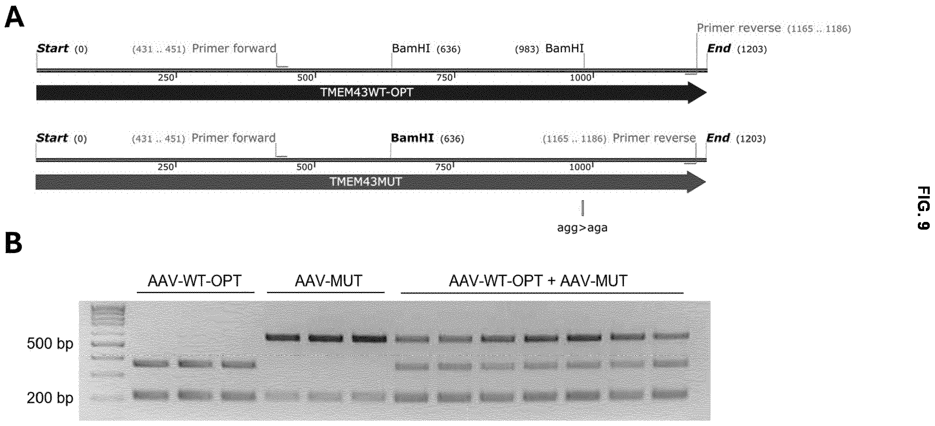

**Figure 9****. WT and mutant TMEM43 expression in the heart of AAV-injected mice.** (A) Illustration of the WT-TMEM43 (blue) and S358L-TMEM43 (red) genes used in AAV-WT-OPT and AAV-MUT, respectively. TMEM43WT-OPT has an additional BamHl restriction site. Forward and reverse primers used for PCR amplification are depicted. (B) Agarose gel resulting from PCR amplification and BamHl digestion of left ventricle cDNA from 16 weeks old mice injected with AAV-WT-OPT, AAV-MUT or both.

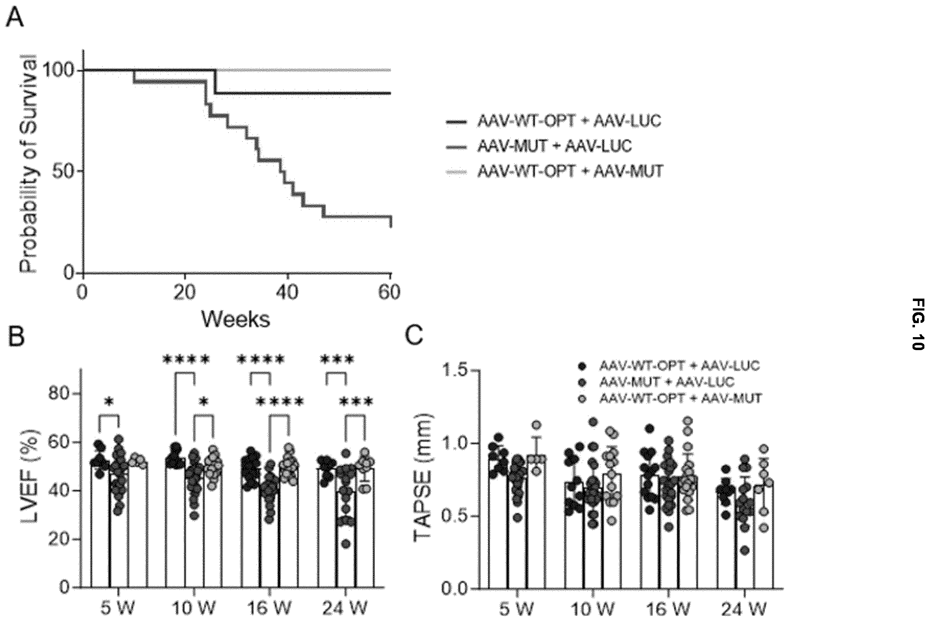

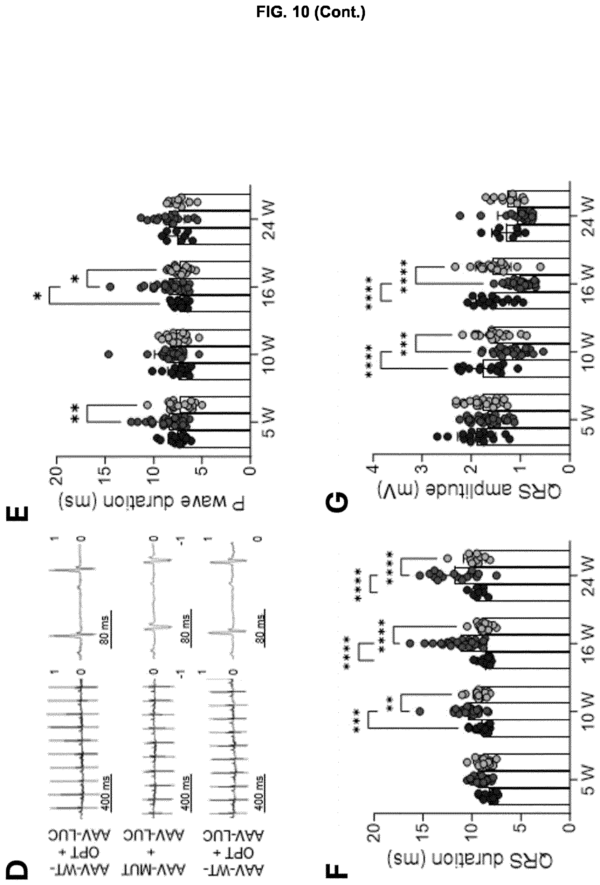

**Figure 10****. AAV-WT-OPT administration improves lifespan, systolic function and** cardiac **conduction defects.** (A) Survival rate of AAV-WT-OPT + AAV-LUC (n=12), AAV-MUT + AAV-LUC (n=19) and AAV-WT-OPT + AAV-MUT (n=13). (B, C) Left ventricular ejection fraction (LVEF, B) and tricuspid annular plane systolic excursion (TAPSE, C) at 5, 10 and 16 weeks after AAV administration. (D-G) Surface ECG at 16 weeks after AAV injection (D). P wave duration (E) and QRS duration and amplitude (F and G) at 5, 10, 16 and 24 weeks after AAV injection. Data are shown as mean ± SD. *p<0.05; **p<0.01; ***p<0.001; ****p<0.0001. Two-way ANOVA was used, followed by Bonferroni post-test.

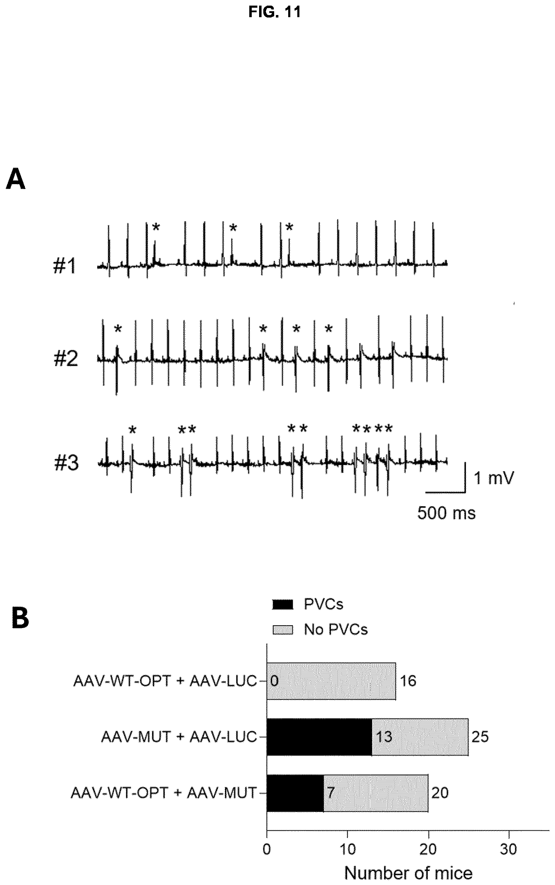

**Figure 11****. Arrhythmic events in AAV-injected mice.** (A) Lead II ECG traces from three AAV-MUT mice at 16 weeks showing premature ventricular contractions (PVCs, asterisks). (B) Number of animals showing PVCs at 5-, 10- or 16-weeks during follow-up.

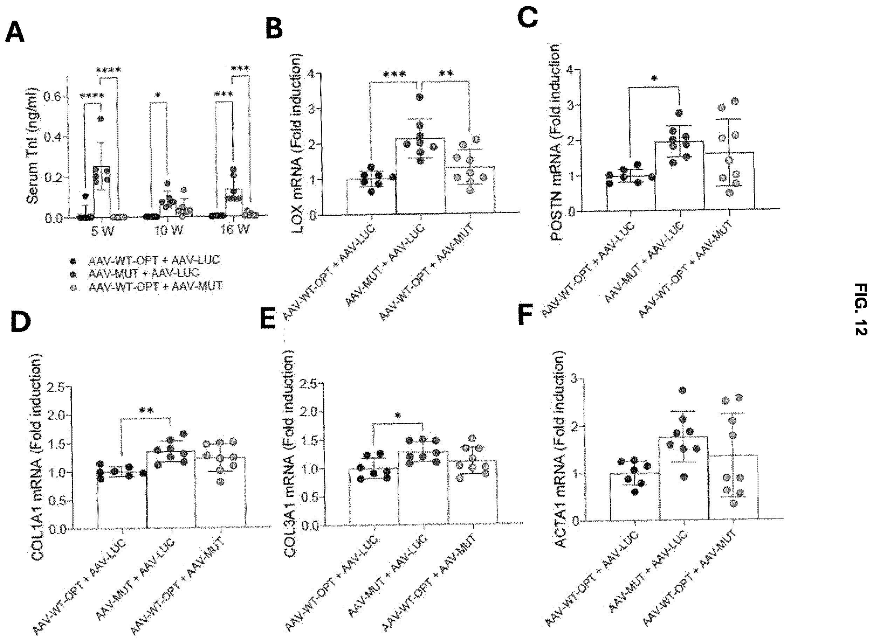

**Figure 12****. AAV-WT-OPT reduces cardiomyocyte necrosis and expression of fibrosis-related genes in mice.** (A) Serum troponin I (Tnl) by ELISA in mice at 5, 10 and 16 weeks of age. (B-F) mRNA expression levels of LOX (B), POSTN (C), COL1A1 (D), COL3A1 (E) and ACTA1 (F) quantified by qRT-PCR in 16 weeks old mouse hearts. Data are shown as mean ± SD. *p<0.05; **p<0.01; ***p<0.001; ****p<0.0001. Two-way ANOVA (A) or one-way ANOVA (B-F) were used, followed by Bonferroni post-test.

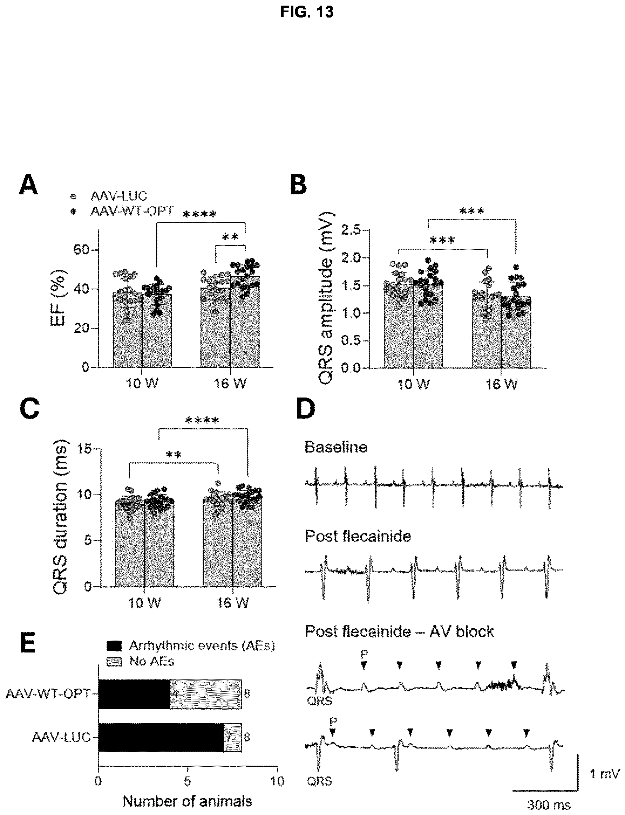

**Figure 13****. Late AAV-WT-OPT treatment in symptomatic ARVC5 mice improves systolic function and reduces arrhythmia susceptibility.** Left ventricular ejection fraction (LVEF, A), QRS duration (B) and QRS amplitude (C) before (10 W) and after (16 W) AAV-WT-OPT or AAV-LUC administration in symptomatic AAV-MUT mice. (D) ECG traces of 16 weeks old mice at baseline, after flecainide, and after flecainide triggering AV blocks. (E) Number of mice showing arrhythmias after flecainide administration. Data are shown as mean ± SD. *p<0.05; **p<0.01; ***p<0.001; ****p<0.0001. Two-way ANOVA was used, followed by Bonferroni post-test.

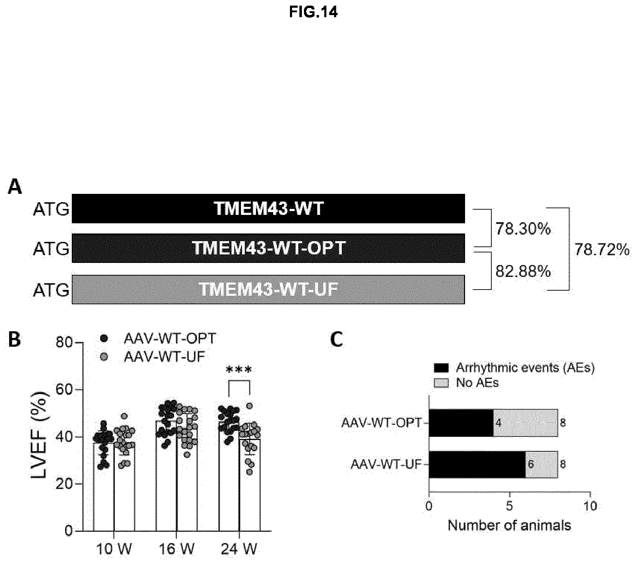

**Figure 14****. Efficacy comparison between AAV-WT-OPT and AAV-WT-UF in the treatment of symptomatic ARVC5 mice.** (A) Sequence identity percentages among the wild type TMEM43 and the two codon-optimized (TMEM43-WT-OPT and TMEM43-WT-UF) cDNAs used for AAV-WT-OPT and AAV-WT-UF, respectively. (B) Left ventricular ejection fraction (LVEF) before (10 weeks) and after treatment with AAV-WT-OPT or AAV-WT-UF (16 and 24 weeks). (C) Number of mice showing arrhythmias after flecainide administration. Data are shown as mean ± SD. *p<0.05; **p<0.01; ***p<0.001; ****p<0.0001. Two-way ANOVA was used, followed by Bonferroni post-test.

### DETAILED DESCRIPTION OF THE INVENTION

Arrhythmogenic right ventricular cardiomyopathy type 5 (ARVC5) is the most aggressive type of ARVC, caused by mutations in TMEM43. Pathologically, the disease is characterized by dilation of the cardiac chambers and fibro-fatty replacement of the myocardium, which results in heart failure and sudden cardiac death. Current therapeutic options are limited and no specific therapies targeting the primary cause of the disease have been proposed.

The present invention is directed to the development of a strategy to overexpress wild type TMEM43 to overcome the detrimental effects of the mutant form. In addition, it also disclosed a systemic delivery of a codon-optimized self-complementary adeno-associated virus (AAV) bearing WT-TMEM43 which improves the disease progression.

In a first aspect, it is disclosed a nucleic acid encoding the human TMEM43 protein, wherein the sequence of the nucleic acid comprises a sequence which has at least 85% identity to the sequence of SEQ ID NO:1 Preferably, the sequence of the nucleic acid comprises SEQ ID NO:5 (WT-TMEM43-OPT), and more preferably the sequence is SEQ ID NO:5. This specific sequence cannot be considered obvious from the wild-type sequence of TMEM43 (WT-TMEM43), since the chosen nucleotides, and no other sequences derived from codon optimization have the activity shown (see Example 7). The sequence is preferably comprised in an expression cassette, more preferably with additional elements such as an operably linked promoter, such as cardiac specific promoter, optionally an enhancer element. SEQ ID NO: 5 has been optimized for expression of TMEM43 in human cells.

In a second aspect, the present disclosure provides an adeno-associated virus (AAV) vector for delivering the WT-TMEM43-OPT transgene of SEQ ID NO: 5 into a cell of a subject, preferably a myocyte.

The term "AAV" is an abbreviation for adeno-associated virus and may be used to refer to the virus itself or derivatives thereof. The AAV vector may include, in addition to a promoter, a regulatory element which modifies expression, e.g., in a manner that provides physiologically relevant expression levels and/or restricts expression to a particular cell type or tissue. In some embodiments, the regulatory element comprises one or more enhancer, a 5' untranslated region (UTR), and a 3' UTR. In some embodiments, the AAV vector may also include at least one polyadenylation signal (e.g., positioned 3' of the WT-TMEM43-OPT transgene of SEQ ID NO: 5). In some embodiments, one or more these transgenes are operably linked to the same single promoter. In some embodiments, each transgene (WT-TMEM43-OPT, SEQ ID NO: 5) is operably linked to a separate promoter. In some embodiments in which multiple transgenes are provided, the AAV vector also includes at least one polyadenylation signal (e.g., positioned 3' of two transgenes expressed from a single promoter or 3' of one or both transgenes expressed from different promoters).

In the present invention, the transgene is WT-TMEM-OPT of SEQ ID NO: 5. The term "AAV" includes AAV serotype 1 (AAV1), AAV serotype 2 (AAV2), AAV serotype 3 (AAV3), AAV serotype 4 (AAV4), AAV serotype 5 (AAV5), AAV serotype 6 (AAV6), AAV serotype 7 (AAV7), AAV serotype 8 (AAV8), AAV serotype 9 (AAV9), serotype rhlO AAV, serotype rh74 AAV, a peptide-displaying AAV (e.g. MYOAAV or AAVmyo) or a pseudotyped rAAV (e.g., AAV2/9, referring an AAV vector with the genome of AAV2 (e.g., the ITRs of AAV2) and the capsid of AAV9). In a preferred embodiment, the adenoviral vector is MYOAAV or AAVmyo (myotube AAV).

In a third aspect of the present disclosure include compositions containing the nucleic acid WT-TMEM43-OPT of SEQ ID NO:5 or an AAV vector comprising thereof (also called therapeutic particles). In a preferred embodiment, the composition is a pharmaceutical composition.

In several embodiments, such compositions may be administered to a subject for gene therapy for a cardiomyopathy, preferably ARVC5. The compositions of the present disclosure may be administered to the subject via different routes. In some embodiments, the composition is administered via intravenous injection into the subject. In some embodiments, the administration of the composition results in expression of WT-TMEM43-OPT in the subject's heart. In various embodiments, the step of administering the composition results in improved cardiac function in the subject, improved cardiac function is represented by an increase in left ventricular ejection fraction (LVEF). In several embodiments, LVEF is measured by echocardiography. In some embodiments, administration results in improved cardiac physiology (e.g., structural features). In a preferred embodiment, the heart disease is arrhythmogenic right ventricular cardiomyopathy (ARVC), preferably arrhythmogenic right ventricular cardiomyopathy type 5 (ARVC5).

Compositions described herein may further comprise a pharmaceutical excipient, buffer, or diluent, and may be formulated for administration to host cell *ex vivo* or *in situ* in an animal, and particularly a human being. Such compositions may further optionally comprise a liposome, a lipid, a lipid complex, a microsphere, a microparticle, a nanosphere, or a nanoparticle, or may be otherwise formulated for administration to the cells, tissues, organs, or body of a subject in need thereof. Such compositions may be formulated for use in a variety of therapies, such as for example, in the amelioration, prevention, and/or treatment of conditions such as peptide deficiency, polypeptide deficiency, peptide overexpression, polypeptide overexpression, including for example, conditions which result in diseases or disorders as described herein.

Formulations comprising pharmaceutically- acceptable excipients and/or carrier solutions are well-known to those of skill in the art, as is the development of suitable dosing and treatment regimens for using the particular compositions described herein in a variety of treatment regimens, including e.g., oral, parenteral, intravenous, intranasal, intra- articular, and intramuscular administration and formulation.

Typically, these formulations may contain at least about 0.1% of the therapeutic agent (e.g., therapeutic AAV particle or preparation) or more, although the percentage of the active ingredient(s) may, of course, be varied and may conveniently be between about 1 or 2% and between about 70% or 90% or more of the weight or volume of the total formulation. Naturally, the amount of therapeutic agent(s) in each therapeutically-useful composition may be prepared in such a way that a suitable dosage will be obtained in any given unit dose of the compound. Factors such as solubility, bioavailability, biological half-life, route of administration, product shelf life, as well as other pharmacological considerations will be contemplated by one skilled in the art when preparing such pharmaceutical formulations. Additionally, a variety of dosages and treatment regimens may be desirable.

In certain circumstances, it will be desirable to deliver the therapeutic particles or preparations in suitably formulated pharmaceutical compositions disclosed herein; either subcutaneously, intracardially, intraocularly, intravitreally, parenterally, subcutaneously, intravenously, intracerebral-ventricularly, intramuscularly, intrathecally, orally, intraperitoneally, by oral or nasal inhalation, or by direct injection to one or more cells (e.g., cardiomyocytes and/or other heart cells), tissues, or organs. In some embodiments, the therapeutic particles or the composition comprising the therapeutic particles of the present invention are delivered systemically via intravenous injection, particularly in those for treating a human. In some embodiments, the therapeutic particles or the composition comprising the therapeutic particles of the present invention (WT-TMEM43-OPT of sequence SEQ ID NO: 5 or AAV comprising thereof) are injected directly into the heart of the subject. Direct injection to the heart may comprise injection into one or more of the myocardial tissues, the cardiac lining, or the skeletal muscle surrounding the heart, e.g., using a needle catheter. In several embodiments, direct injection to human heart is preferred, for example, if delivery is performed concurrently with a surgical procedure or interventional procedure whereby access to the heart is improved. In some embodiments, the interventional procedure includes any procedure wherein coronary or pulmonary perfusion is altered. In some embodiments, the interventional procedure includes one or more of percutaneous administration, catheterization, or coronary retroperfusion.

A pharmaceutical composition or medicament includes a pharmacologically effective amount of at least one of the therapeutic and optionally one or more pharmaceutically acceptable excipients. Pharmaceutically acceptable excipients (excipients) are substances other than the Active Pharmaceutical ingredient (API, therapeutic product) that are intentionally included in the drug delivery system. Excipients do not exert or are not intended to exert a therapeutic effect at the intended dosage. Excipients may act to a) aid in processing of the drug delivery system during manufacture, b) protect, support or enhance stability, bioavailability or patient acceptability of the API, c) assist in product identification, and/or d) enhance any other attribute of the overall safety, effectiveness, of delivery of the API during storage or use. A pharmaceutically acceptable excipient may or may not be an inert substance.

Excipients include, but are not limited to: absorption enhancers, anti- adherents, anti-foaming agents, anti-oxidants, binders, buffering agents, carriers, coating agents, colors, delivery enhancers, delivery polymers, dextran, dextrose, diluents, disintegrants, emulsifiers, extenders, fillers, flavors, glidants, humectants, lubricants, oils, polymers, preservatives, saline, salts, solvents, sugars, suspending agents, sustained release matrices, sweeteners, thickening agents, tonicity agents, vehicles, water-repelling agents, and wetting agents.

The pharmaceutical compositions can contain other additional components commonly found in pharmaceutical compositions. Such additional components can include, but are not limited to anti-pruritic s, astringents, local anesthetics, or anti-inflammatory agents (e.g., antihistamine, diphenhydramine, etc.).

The carrier can be, but is not limited to, a solvent or dispersion medium containing, for example, water, ethanol, polyol (for example, glycerol, propylene glycol, and liquid polyethylene glycol), and suitable mixtures thereof. A carrier may also contain adjuvants such as preservatives, wetting agents, emulsifying agents, and dispersing agents. A carrier may also contain isotonic agents, such as sugars, polyalcohols, sodium chloride, and the like into the compositions.

Pharmaceutically acceptable refers to those properties and/or substances which are acceptable to the subject from a pharmacological/toxicological point of view. The phrase pharmaceutically acceptable refers to molecular entities, compositions, and properties that are physiologically tolerable and do not typically produce an allergic or other untoward or toxic reaction when administered to a subject. In some embodiments, a pharmaceutically acceptable compound is approved by a regulatory agency for use in animals and more particularly in humans.

In a fourth aspect, the present disclosure includes pharmaceutical compositions comprising the sequence of WT-TMEM43-OPT of SEQ ID NO:5 or an AAV vector comprising thereof (AAV-WT-OPT) for use in therapy or for use in the treatment of a disease.

In an alternative embodiment, it is disclosed the use of pharmaceutical compositions comprising the sequence of WT-TMEM43-OPT of SEQ ID NO:5 or an AAV vector comprising thereof (AAV-WT-OPT) for the manufacture of a medicament.

In an alternative embodiment, it is disclosed a method of treatment comprising the administration of a pharmaceutical compositions comprising the sequence of WT-TMEM43-OPT of SEQ ID NO:5 or an AAV vector comprising thereof (AAV-WT-OPT).

In a fifth aspect, the present disclosure is directed to pharmaceutical compositions comprising the sequence of WT-TMEM43-OPT of SEQ ID NO:5 or an AAV vector comprising thereof (AAV-WT-OPT) for use in the treatment of a cardiomyopathy in a subject. In a preferred embodiment, the cardiomyopathy a disease linked to TMEM43 mutations and/or a disease linked to TMEM43 downregulation. In a preferred embodiment, the disease linked to TMEM43 mutations is selected from Emery-Dreifuss-related myopathy and autosomal dominant auditory neuropathy spectrum disorder or an arrhythmogenic cardiomyopathy. In a more preferred embodiment, the disease is an arrhythmogenic right ventricular cardiomyopathy (ARVC), more preferably Arrhythmogenic right ventricular cardiomyopathy type 5 (ARVC5). In another preferred embodiment, the disease linked to TMEM43 downregulation is selected from cardiac hypertrophy and sepsis-induced cardiomyopathy.

In an alternative embodiment, the present disclosure is directed to the use of a pharmaceutical composition comprising the sequence of WT-TMEM43-OPT of SEQ ID NO:5 or an AAV vector comprising thereof (AAV-WT-OPT) for the manufacture of a medicament for treating a cardiopathy. In a preferred embodiment, the cardiomyopathy a disease linked to TMEM43 mutations and/or a disease linked to TMEM43 downregulation. In a preferred embodiment, the disease linked to TMEM43 mutations is selected from Emery-Dreifuss-related myopathy and autosomal dominant auditory neuropathy spectrum disorder or an arrhythmogenic cardiomyopathy. In a more preferred embodiment, the disease is an arrhythmogenic right ventricular cardiomyopathy (ARVC), more preferably Arrhythmogenic right ventricular cardiomyopathy type 5 (ARVC5). In another preferred embodiment, the disease linked to TMEM43 downregulation is selected from cardiac hypertrophy and sepsis-induced cardiomyopathy.

In an alternative embodiment, the present disclosure is directed to a method for the treatment of a cardiomyopathy comprising the administration of a therapeutically effective amount of pharmaceutical composition comprising the sequence of WT-TMEM43-OPT of SEQ ID NO:5 or an AAV vector comprising thereof (AAV-WT-OPT) as described above. In a preferred embodiment, the cardiomyopathy a disease linked to TMEM43 mutations and/or a disease linked to TMEM43 downregulation. In a preferred embodiment, the disease linked to TMEM43 mutations is selected from Emery-Dreifuss-related myopathy and autosomal dominant auditory neuropathy spectrum disorder or an arrhythmogenic cardiomyopathy. In a more preferred embodiment, the disease is an arrhythmogenic right ventricular cardiomyopathy (ARVC), more preferably Arrhythmogenic right ventricular cardiomyopathy type 5 (ARVC5). In another preferred embodiment, the disease linked to TMEM43 downregulation is selected from cardiac hypertrophy and sepsis-induced cardiomyopathy.

In the above embodiments, the administration results in expression of a therapeutically effective amount of wild type human TMEM43, thereby treating the cardiomyopathy.

In the above embodiments, the subject recipient of the composition is a mammal. In a more preferred embodiment is a human.

Preferably, the administration of the pharmaceutical composition is via intravenous injection. However, the administration may be adapted depending on the treatment of medical decision in each case.

Additionally, provided herein are compositions, as well as therapeutic and/or diagnostic kits that include one or more of the disclosed compositions, formulated with one or more additional ingredients, or prepared with one or more instructions for their use. As used herein, the term "kit" may be used to describe variations of the portable, self-contained enclosure that includes at least one set of components to conduct one or more of the diagnostic or therapeutic methods of the invention.

Kits comprising one or more of the disclosed sequences of WT-TMEM-OPT of SEQ ID NO: 5 or AAV vectors comprising thereof (as well as one or more virions, viral particles, transformed host cells or pharmaceutical compositions comprising such vectors); and instructions for using such kits in one or more therapeutic, diagnostic, and/or prophylactic clinical embodiments are also provided. Such kits may comprise one or more reagents, restriction enzymes, peptides, therapeutics, pharmaceutical compounds, or means for delivery of the composition(s) to host cells, or to an animal (e.g., syringes, injectables, and the like). Depending on the embodiment, kits include those for treating, preventing, or ameliorating the symptoms of a disease, deficiency, dysfunction, and/or injury, or may include components for the large-scale production of the viral vectors themselves.

One or more of the components of a kit can be provided in one or more liquid or frozen solvents. The solvent can be aqueous or non-aqueous. The formulation in the kit can also be provided as dried powder(s) or in lyophilized form that can be reconstituted upon addition of an appropriate solvent.

In some embodiments, a kit comprises a label, marker, package insert, bar code and/or reader indicating directions of suitable usage of the kit contents. In some embodiments, the kit may comprise a label, marker, package insert, bar code and/or reader indicating that the kit contents may be administered in accordance with a certain dosage or dosing regimen to treat a subject.

In addition, a kit may also contain various reagents, including, but not limited to, wash reagents, elution reagents, and concentration reagents. Such reagents may be readily selected from among the reagents described herein, and from among conventional concentration reagents.

Also described herein in a sixth aspect, is a method of increasing expression of human TMEM43 (WT-TMEM) of SEQ ID NO: 2 in a target cell, comprising contacting a target cell with a plurality of particles comprising a nucleic acid expression construct comprising a functional human TMEM43 coding sequence as set forth in SEQ ID NO: 1 and wherein said contacting results in the target cell increasing expression of functional human TMEM43. In a preferred embodiment, the sequence is operably linked to a promoter and optionally an enhancer element. In a more preferred embodiment, these sequences are integrated in an AAV. The method may be performed *in vivo, ex vivo* or *in vitro.* In a preferred embodiment, the promoter is the cardiac specific promoter cTNT, preferably as set forth in SEQ ID NO: 21. Additionally, the sequence additionally comprises the SV40 late polyA signal, preferably, as set forth in SEQ ID NO: 22.

The invention also discloses a nucleic acid cassette comprising a functional human TMEM43 coding sequence as set forth in SEQ ID NO: 1 operable linked to the cTnT promoter of SEQ ID NO: 21 and the SV40 late polyA signal, for use in a method for treating a disease. The invention also discloses the nucleic acid cassette comprising a functional human TMEM43 coding sequence as set forth in SEQ ID NO: 1 operable linked to the cTnT promoter of SEQ ID NO: 21 and the SV40 late polyA signal, for use in a method for treating a cardiac disease. In a preferred embodiment, the cardiac disease is a disease linked to TMEM43 mutations and/or a disease linked to TMEM43 downregulation. In a preferred embodiment, the disease linked to TMEM43 mutations is selected from Emery-Dreifuss-related myopathy and autosomal dominant auditory neuropathy spectrum disorder or an arrhythmogenic cardiomyopathy. In a more preferred embodiment, the disease is an arrhythmogenic right ventricular cardiomyopathy (ARVC), more preferably Arrhythmogenic right ventricular cardiomyopathy type 5 (ARVC5). In another preferred embodiment, the disease linked to TMEM43 downregulation is selected from cardiac hypertrophy and sepsis-induced cardiomyopathy.

In some embodiments, the contacting is *in vivo.* In some embodiments, the method is for the treatment of arrhythmogenic cardiomyopathy, preferably arrhythmogenic right ventricular cardiomyopathy (ARVC) and more preferably arrhythmogenic right ventricular cardiomyopathy type 5 (ARVC5).

The compositions of the present disclosure may include therapeutic particles of the invention (WT-TMEM43-OPT of SEQ ID NO: 5 and/or an AAV vector comprising SEQ ID NO: 5 and/or the overexpression of SEQ ID NO:2) or preparations, and/or AAV vectors, either alone or in combination with one or more additional therapeutic agents.

If desired, the therapeutic particles may be administered in combination with other agents as well, such as, e.g., proteins or polypeptides or various pharmaceutically active agents. The amount of compositions containing the disclosed therapeutic particles and the additional therapeutic agent, and the time of administration of such compositions, will be within the purview of the skilled artisan having benefit of the present teachings. It is likely, however, that the administration of therapeutically- effective amounts of the compositions of the present disclosure may be achieved by co-administration or separate administration.

The disclosure herein of any particular feature, aspect, method, property, characteristic, quality, attribute, element, or the like in connection with an embodiment can be used in all other embodiments set forth herein. Accordingly, it should be understood that various features and aspects of the disclosed embodiments can be combined with or substituted for one another in order to form varying modes of the disclosed inventions. Thus, it is intended that the scope of the present inventions herein disclosed should not be limited by the particular disclosed embodiments described above. Moreover, while the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents, and alternatives falling within the spirit and scope of the various embodiments described and the appended claims.

Terms and phrases used in this application, and variations thereof, especially in the appended claims, unless otherwise expressly stated, should be construed as open ended as opposed to limiting. As examples of the foregoing, the term "including" should be read to mean 'including, without limitation,' 'including but not limited to,' or the like; the term "comprising" as used herein is synonymous with 'including,' 'containing,' or 'characterized by,' and is inclusive or open-ended and does not exclude additional, unrecited elements or method steps; the term 'having' should be interpreted as 'having at least;' the term "includes" should be interpreted as `includes but is not limited to;' the term 'example' is used to provide exemplary instances of the item in discussion, not an exhaustive or limiting list thereof; and use of terms like 'preferably,' 'preferred,' 'desired,' or 'desirable,' and words of similar meaning should not be understood as implying that certain features are critical, essential, or even important to the structure or function, but instead as merely intended to highlight alternative or additional features that may or may not be utilized in a particular embodiment. In addition, the term "comprising" is to be interpreted synonymously with the phrases "having at least" or "including at least". When used in the context of a process, the term "comprising" means that the process includes at least the recited steps but may include additional steps. When used in the context of a compound, composition or device, the term "comprising" means that the compound, composition, or device includes at least the recited features or components, but may also include additional features or components. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

### EXAMPLES

The invention is defined by the claims. Any subject-matter outside the scope of the claims is provided for Information purposes only.

### Methods

### Mice

TMEM43wt and TMEM43mut mice, in the C57BL/6J background, express human wild type (SEQ ID NO: 2) and human TMEM43-S358L (SEQ ID NO: 4) respectively specifically in cardiomyocytes under the control of the α-myosin heavy chain promoter (MHC), as described in Padrón-Barthe L *et al.,* 2019. Double transgenic mice were obtained by crossing TMEM43wt and TMEM43mut mice. Genotyping was performed by Sanger sequencing after PCR amplification of human TMEM43 with the following primers: human TMEM43 forward 5'-TCCCAAGTATCCAGAGGTGGGAGACT-3' (SEQ ID NO: 7) and human TMEM43 reverse 5'- AGGCCGGCAATGAGGAGGG-3' (SEQ ID NO: 8). WT littermates were used as controls. Male and female mice were used throughout the study.

Mice were housed in an air-conditioned room with a 12-hour light/dark cycle and free access to water and chow. Mice were sacrificed in a carbon dioxide chamber. After sacrifice, tibial length was measured, and hearts were weighed. Power calculations were carried out to determine the necessary number of animals based on prior data (p=0.05; power=80%). All procedures were approved by the Ethics Committees of the CNIC and the Regional Government of Madrid (PROEX191.5/22).

### Echocardiography

Transthoracic echocardiography was performed in mice aged 5, 10, 16 and 24 weeks. Mice were placed on a heating pad and kept under light anesthesia with isoflurane administered via nose cone. The anesthesia was adjusted to obtain a target heart rate of 500±50 bpm. Cardiac function, chamber dilation, and wall thickness were analyzed from two-dimensional (2D) and M-mode (MM) acquisitions. Image acquisitions were carried out by a blinded operator using a high-frequency ultrasound system with a 30-MHz probe (Vevo 2100, Visualsonics). LV ejection fraction (LVEF) and LV end-diastolic volume (LVEDV) measurements were obtained from the long axis view, and LV posterior wall thickness in diastole (LVPWd) values were analyzed in the short axis view. Right ventricular (RV) systolic function was assessed indirectly from the tricuspid annular plane systolic excursion (TAPSE), estimated from maximum lateral tricuspid annulus movement obtained from a 2D 4-chamber apical view. Images were analyzed off-line by an expert blinded to genotype and treatment using the Vevo 2100 Workstation software.

### Electrocardiograms and flecainide administration

Mice were anesthetized with isoflurane via nose cone as for echocardiography. Surface electrocardiograms (ECGs) were obtained by using bipolar limb leads (leads I, II, and III) and unipolar limb leads (leads aVR, aVL, and aVF) for 90 seconds. Recordings were acquired and analyzed by a blinded operator using Acqknowledge 4.1.1 for MP36R (BIOPAC Systems, Inc.). Mean values of P-wave duration and amplitude, R-wave and S-wave amplitudes and QRS duration and amplitude were calculated from 3 non-consecutive beats from lead II. Mice that exhibited premature ventricular contractions (PVCs) in at least one 90-second ECG recording during follow-up were quantified. For experiments with flecainide, 90 seconds baseline ECG recordings were obtained prior to fleicainide administration via intraperitoneal injection (40 mg/kg). ECG recordings continued for ~5 minutes post-administration.

### Cell culture and immunoprecipitation

TMEM43 coimmunoprecipitation (co-IP) experiments were performed in the P19 cell line. P19 cells were transfected with both HA-tagged and GFP-tagged TMEM43 WT and S358L constructs using Lipofectamine 2000 (11668019, Thermo Fisher Scientific). Cells were lysed with TBS buffer supplemented with 1% IGEPAL, 5 mM EDTA, 5 mM MgCl₂ and protease and phosphatase inhibitors (5892791001 and 04906845001, respectively, from Roche) for 30 minutes at RT. After centrifugation, the supernatants were precleared with Protein G Dynabeads (10003D, Thermo Fisher Scientific). Proteins were immunoprecipitated with Protein G Dynabeads coupled with a rabbit anti-GFP antibody (632592, Takara Bio), rotating at 4°C overnight. Beads were washed three times with 0.05% IGEPAL lysis buffer and five times with lysis buffer without added detergent. Proteins were eluted from beads by boiling for 5 minutes at 95°C in Laemmli sample buffer. Input, supernatant and output samples from the co-IP were resolved by SDS-PAGE.

For TMEM43 codon-optimization experiments, HL1 cells were transfected with plasmids carrying a chicken cardiac troponin T promoter (cTnT) driving GFP, human codon-optimized TMEM43 or human wild-type TMEM43 expression using jetPRIME transfection reagent (POL101000046, Polyplus).

### Western blots

LV myocardium samples or transfected HL1 cells were homogenized by manual grinding in RIPA buffer in the presence of protease and phosphatase inhibitors (5892791001 and 04906845001, respectively, from Roche). Lysates were separated in SDS-PAGE gels, transferred to polyvinylidene fluoride (PVDF) membranes, and blocked with 3% non-fat dry milk in 1X TBS for 1 hour. Membranes were incubated with primary antibodies overnight, followed by appropriate horseradish peroxidase (HRP)-labelled secondary antibodies (antimouse P044701, and anti-rabbit P044801-2, Dako). HRP activity was detected using Radiance ECL chemiluminiscent HRP substrate (AC2204, Azure Biosystems). Primary antibodies were as follows: TMEM43 (ab184164, Abcam), GFP (11814460001, Roche), HA (11583816001, Roche) and vinculin (V4505; Sigma). Blot images were obtained and quantified with Invitrogen iBright CL750 Imaging System and analysis software (Thermo Fisher Scientific).

### RNA isolation and qRT-PCR

LV samples were snap-frozen in liquid nitrogen right after sacrifice. Total RNA was isolated using TRIzol reagent (15596026, Thermo Fisher Scientific). First-strand cDNA was synthesized using 100 ng of total RNA and a High-Capacity cDNA Reverse Transcription Kit (4368814, Thermo Fisher Scientific). Quantitative reverse-transcribed PCR (qRT-PCR) was carried out in a QuantStudio 5 real-time PCR thermocycler (Thermo Fisher Scientific) using SYBR Green (4367659, Thermo Fisher Scientific) for double-stranded DNA detection. Primers used for qRT-PCR are: LOX forward 5'- GCTGCGGAAGAAAACTGC-3' (SEQ ID NO: 9), LOX reverse 5'- CCTTGGTTCTTCACTCTTTGC-3' (SEQ ID NO: 10), POSTN forward 5'- AACGTCTGTGCCCTCCAG-3' (SEQ ID NO: 11), POSTN reverse 5'-AGCCTTTCATCCCTTCCATT-3' (SEQ ID NO: 12), COL1A1 forward 5'-GTGCCACTCTGACTGGAAGA-3' (SEQ ID NO: 13), COL1A1 reverse 5'-CTGACCTGTCTCCATGTTGC-3' (SEQ ID NO: 14), COL3A1 forward 5'-CACCCTTCTTCATCCCACTC-3' (SEQ ID NO: 15), COL3A1 reverse 5'-ATGTCATCGCAAAGGACAGA-3' (SEQ ID NO: 16), ACTA1 forward 5'-GACCACAGCTGAACGTGAGA-3' (SEQ ID NO: 17), ACTA1 reverse 5'-TGTTGTAGGTGGTCTCATGGAT-3' (SEQ ID NO: 18), Luciferase forward 5'-TATCATGGCCTCGTGAAATCC-3' (SEQ ID NO: 19), Luciferase reverse 5'-TCCTGGGTCCGATTCAATAAAC-3' (SEQ ID NO: 20). Results were analyzed with the LinReg PCR software and normalized to β-actin expression levels.

### Histological analysis, immunohistochemistry and immunofluorescence

Hearts were fixed in 4% paraformaldehyde (PFA) in PBS for 48 hours, washed in PBS, dehydrated, included in paraffin, and sliced in 5 µm sections. The collagen content was analyzed using Masson's trichrome protocol. For immunofluorescence, heart sections were subjected to sodium citrate antigen retrieval, permeabilized with 0.1% Tween-20/PBS for 20 min, and blocked with blocking solution (5% goat serum, 2% MgCl2, 3% BSA in 0.3% Tween-20/PBS). Sections were incubated with anti-TMEM43 (ab184164, Abcam) diluted 1:100, followed by secondary antibody Alexa 568-labeled goat anti-rabbit (A-11011, Thermo Fisher Scientific) diluted 1:200, and WGA Alexa Fluor 488 conjugate (W11261, Thermo Fisher Scientific) diluted 1:2000. Nuclei were stained with DAPI (D1306, Thermo Fisher Scientific). Samples were mounted in Vectashield mounting medium (H-1000, Vector Laboratories).

Immunofluorescence images were acquired with a Leica SP5 confocal microscope, a HCX PL APO CS 10x 0.4 dry objective, and Leica LAS-AF 2.7.3 software. Images for immunohistochemistry were digitized with NanoZoomer-2.0RS (Hamamatsu). All images were analyzed off-line with Fiji and brightness and contrast were linearly adjusted.

### Brillouin spectroscopy

Brillouin spectra were acquired with a home modified Olympus BX51 reflected light microscope with a DPSS laser at 532 nm (SpectraPhysics) and a light power below 3.5 mW on the sample coupled to a 3 + 3 TandemFabry-Perot spectrometer (J. Sandercock, Table Stable Ltd.) was used (Rioboó RJJ Gontán et al. Spectroscopy: From Biomedical Research to New Generation Pathology Diagnosis. Int J Mol Sci 22, 8055 (2021); Villalba-Orero M et al. Assessment of myocardial viscoelasticity with Brillouin spectroscopy in myocardial infarction and aortic stenosis models. Sci Rep 11, 21369 (2021)).

An Olympus MPlan 10X objective with numerical aperture of 0.25 was used to focus the incident light beam and to collect the scattered light simultaneously. Measurements were performed on dried samples to avoid having to account for water content variability. The tip of the heart apex was carefully sliced to allow the entry of agarose into the ventricular lumen and samples were embedded in agarose (0.2 g/10 mL distilled water) and sliced into 500 µm sections using a vibratome (Campdem Instruments). A cover slip was placed on top of the sample without sealing. Transparent nail polish was applied on the sides to fix the cover slip. Samples were dried out at room temperature for 5 h and stored at 4°C overnight. Brillouin frequency shift, its error and the Half Width Half Maximum (HWHM) were obtained from the Brillouin spectra by a non-linear least squares fitting procedure using a Pseudo-Voigt function and a cubic polynomic background in MATLAB environment. Lab temperature monitoring, displacement of the Märzhäuser Wetzler automated table and the GHOST multichannel analyzer were controlled with our own developed software using LABVIEW 4 platform.

### AAV vector production and delivery

Codon-optimization of human TMEM43 was performed using VectorBuilder design studio. Self-complementary AAV vectors AAV-WT-OPT, AAV-MUT and AAV-LUC expressing optimized human WT-TMEM43, S358L-TMEM43 and luciferase, respectively, were constructed by VectorBuilder. Viral Vector Unit of the Spanish Centre of Cardiovascular Diseases (CNIC) produced the recombinant AAVs with the AAVMYO myotropic capsid (Weinmann, J. et al. Identification of a myotropic AAV by massively parallel in vivo evaluation of barcoded capsid variants. Nature Communications 2020 11:1 11, 1-12 (2020)) using a modified Rep/Cap vector. Briefly, the cap gene from the AAV9 variant in the pAAV2/9n helper plasmid (gift from James M. Wilson, addgene #112865) was replaced by digestion and ligation with a 3014-bp Swal+Sbfl fragment encoding the AAVMYO VP1 gene (obtained by synthesis from the public genbank sequence deposited under accession: MN365014).

For viral administration, neonatal mice at postnatal day 1 (P1) were lightly anaesthetized with ice and 30 µL of the viral preparation including 5e10 viral particles (VP) were injected through the temporal vein in a single injection (1.7e13 VP/kg). Neonates recovered on a heating mat before placing them back together with the dams.

For experiments involving symptomatic adult mice, a subgroup of AAV-MUT mice was randomized into two subgroups based on left ventricular ejection fraction (LVEF) and QRS amplitude. Mice without signs of disease were excluded. These subgroups were then treated at 10 weeks of age with 1.7e13 VP/kg of either AAV-LUC or AAV-WT-OPT via the femoral vein.

### Statistical analysis

Statistical analysis was performed using GraphPad Prism version 10.0.0 (GraphPad Software). All datasets were analyzed for statistical significance by regular one-way or two-way ANOVA followed by Bonferroni's post-test for multiple comparisons, as indicated in the figure legends. Survival curves were compared by the log-rank (Mantel-Cox) test. Differences were considered statistically significant at p<0.05. Data are presented as mean ± standard deviation (SD).

### Example 1. WT-TMEM43 overexpression in the heart improves cardiac contractility, reduces electrophysiological abnormalities and extends lifespan

It was previously shown by *in silico* models, that WT-TMEM43 (SEQ ID NO: 2) and S358L-TMEM43 (SEQ ID NO: 4) can form heterodimers. To confirm the models experimentally, it was conducted IP experiments using tagged proteins to increase specificity. It was found that GFP-tagged S385L-TMEM43 co-immunoprecipitated with HA-tagged S358L-TMEM43 as well as with HA-tagged WT-TMEM43 (**Figure 1A**). This result validated the *in silico* models and led to hypothesize that the overexpression of WT-TMEM43 might quench the deleterious dominant effects of the mutant protein by forming a heterodimer.

In order to explore whether WT-TMEM43 overexpression could reduce the toxicity of S358L-TMEM43, we crossed the ARVC5 mouse model overexpressing human S358L-TMEM43 (TMEM43mut) with mice overexpressing human WT-TMEM43 (TMEM43wt mice) to generate a double transgenic mouse line that overexpressed both forms of the human protein in a cardiac-specific manner. The functional effects were evaluated through echocardiography and ECG over 24 weeks and double transgenic mice were compared to TMEM43mut, TMEM43wt and WT littermates.

The lifespan of double transgenic mice increased significantly by more than 10 weeks compared to mice overexpressing the mutant TMEM43, with a median lifespan of 34.6 vs 23.9 weeks, respectively (**Figure 1B**). Echocardiography analysis revealed improved LV contraction (LVEF) and reduced dilation (LVESV) in double transgenic mice (**Figure 1C** and **1D****,** respectively), while LV wall thickness remained unaltered in all the groups (**Figure 1E**). RV contractility was also improved in double transgenic mice, without any significant deterioration observed until 24 weeks of age (**Figure 1F**). In addition, double transgenic mice exhibited reduced heart weight to tibial length ratio compared to TMEM43mut mice (**Figure 1G**).

Overexpression of WT-TMEM43 also improved ECG abnormalities caused by the mutant protein (**Figure 2A-D**). In contrast to the progressive P-wave prolongation observed in TMEM43mut mice, double transgenic mice exhibited normal P-wave duration (**Figure 2B**). Similarly, double transgenic mice showed reduced QRS duration and increased QRS amplitude compared to TMEM43mut mice at both 16 and 24 weeks of age (**Figure 2C, 2D**). Furthermore, the proportion of mice showing premature ventricular contractions (PVCs), a common pathological manifestation in ARVC5 patients, was reduced to normal WT levels in double transgenic mice (-12.5%), compared to TMEM43mut mice (-35%) (**Figure 3A, 3B**). In line with these results, the mislocalization of Cx43 observed in TMEM43mut mice was also partially prevented in double transgenic mice (**Figure 4**).

### Example 2. WT-TMEM43 overexpression reduces myocardial fibrosis

As demonstrated in Example 1, ARVC5 mice develop extensive myocardial fibrosis as a result of cardiomyocyte death, evidenced by an increase in circulating cardiac troponin I (Tnl) levels (Padrón-Barthe, L et al. Severe Cardiac Dysfunction and Death Caused by Arrhythmogenic Right Ventricular Cardiomyopathy Type 5 Are Improved by Inhibition of Glycogen Synthase Kinase-3β. Circulation 140, 1188-1204 (2019)). As shown in **Figure 5A****,** double transgenic mice showed a delayed rise in circulating Tnl compared to mutant mice, in agreement with the delayed onset of the disease. Moreover, double transgenic mice presented reduced expression of fibrosis markers at week 16, including lysyl oxidase (LOX) which is responsible for collagen and elastin crosslinking, periostin (POSTN), and collagens 1 and 3 (COL1A1, COL3A1), together with a decrease of the cardiac stress marker ACTA1 (**Figure 5B-F**). Masson trichrome staining confirmed a reduced fibrotic content in the heart of double transgenic mice compared to TMEM43mut mice at both 16 and 24 weeks (**Figure 6A-E**). Consistent with these results, TMEM43mut mice showed an increased Brillouin frequency shift, indicating increased stiffness of the myocardial tissue compared to double transgenic, wild type and TMEM43wt mice (**Figure 6F**). Together, these results demonstrate that WT-TMEM43 overexpression reduces cardiomyocyte death, fibrotic replacement and myocardial stiffness, leading to improved cardiac function.

### Example 3. WT-TMEM43 expression is enhanced by codon optimization

Following the positive impact of WT-TMEM43 overexpression in the ARVC5 mice, it was developed a gene therapy tool based on AAVs carrying WT-TMEM43 to further explore the therapeutic potential of this approach. To enhance vector efficiency, it was first investigated the influence of cDNA codon optimization on the expression of human WT-TMEM43 (SEQ ID NO: 2). This process included substituting rare or non-preferred codons in the original cDNA sequence with synonymous codons that are more frequently used by the host, likely improving mRNA stability and protein translation efficiency without altering the encoded protein's amino acid sequence. HL1 cells were transfected with control (GFP), human TMEM43 (TMEM43WT, SEQ ID NO: 1), and codon-optimized human TMEM43 (TMEM43WT-OPT) constructs, and protein expression was assessed through western blot. Our results revealed a positive impact of codon optimization on TMEM43 protein expression (**Figure 7A and 7B**), prompting the utilization of this optimized sequence for the development of a self-complementary myotropic AAV. This vector expresses codon-optimized TMEM43 specifically in the cardiac tissue under the control of the cTnT promoter, hereafter referred to as AAV-WT-OPT (**Figure 7C**).

### Example 4. AAV-WT-OPT prevents systolic dysfunction and EGC abnormalities caused by mutant TMEM43

To determine the efficacy of the gene therapy approach and ensure a similar ratio of WT to mutant human TMEM43, we used systemic delivery of myotropic AAVs in neonates through the temporal vein and followed the animals for 24 weeks (**Figure 8A**). Each neonate received a total of 5e10 VP distributed as follows: 2.5e10 VP of AAV-WT-OPT + 2.5e10 VP of AAV carrying luciferase (AAV-LUC), 2.5e10 VP of AAV carrying S358L-TMEM43 (AAV-MUT) + 2.5e10 VP of AAV-LUC, or 2.5e10 VP of AAV-WT-OPT + 2.5e10 VP of AAV-MUT.

Western blot and PCR analyses revealed sustained TMEM43 overexpression in mice infected with AAV-WT-OPT and/or AAV-MUT (**Figure 8B****,** **Figure 9**). Similarly, mice infected or co-infected with AAV-LUC expressed high levels of luciferase mRNA, as determined by qRT-PCR (**Figure 8C**). Immunofluorescence analysis of human TMEM43 showed overexpression of the protein in 10-30% of cardiomyocytes (**Figure 8D-K**), indicating wide biodistribution of the viral infection and cell type-specific protein expression.

Consistent with the TMEM43mut transgenic mice, mice transduced with AAV-MUT + AAV-LUC showed reduced lifespan (**Figure 10A**) and a progressive decline in LVEF as assessed by echocardiography (**Figure 10B**). This cardiac function impairment and lifespan decline were successfully prevented in mice co-infected with AAV-WT-OPT + AAV-MUT, providing additional support for the therapeutic potential of WT-TMEM43 overexpression. Right ventricular function showed similar trends, although the differences were milder and did not reach statistical significance (**Figure 10C**). AAV-MUT mice also developed ECG abnormalities, including increased P-wave duration, progressive widening of the QRS and a gradual decline of the QRS amplitude, mirroring the pathological changes observed in our TMEM43mut transgenic mice. Remarkably, co-administration of AAV-WT-OPT proved sufficient to prevent all these ECG abnormalities (**Figure 10D-G**). In addition, the proportion of mice showing PVCs during follow-up is reduced in AAV-WT-OPT + AAV-MUT co-infected mice compared to AAV-MUT mice (**Figure 11**).

### Example 5. AAV-WT-OPT prevents cardiomyocyte necrosis and upregulation of fibrosis-related genes

AAV-MUT mice also showed a significant increase in circulating cardiac troponin I levels, albeit to lower levels than TMEM43mut transgenic mice (**Figure 12A**). This cardiomyocyte death was accompanied by mild but significant increases in the expression of genes related to fibrosis, including LOX, POSTN and collagens 1 and 3, and the cardiac stress marker ACTA1 (**Figure 12B-F**). Importantly, co-infection with AAV-WT-OPT + AAV-MUT strongly reduced cardiomyocyte death and partially prevented the induction of fibrosis and stress genes (**Figure 12A-F**). Collectively, these findings support that AAV-WT-OPT gene therapy can prevent cardiomyocyte necrosis and the upregulation of fibrosis-related genes, improving cardiac contraction and reducing ECG abnormalities, in agreement with the results obtained in the double transgenic mice.

### Example 6. Treatment of symptomatic ARVC5 mice with AAV-WT-OPT improves systolic function and reduces arrhythmia susceptibility

It has been demonstrated the potential of WT-TMEM43 overexpression as a preventive therapeutic strategy. To investigate whether late administration of the therapy could also ameliorate the ARVC5 phenotype, it was administered AAV-WT-OPT to adult AAV-MUT mice after overt symptoms of the disease were already evident (10 weeks of age), including low LVEF, reduced QRS amplitude, and increased QRS duration. These mice were randomized into two groups with matched median LVEF and QRS amplitude and the same dose of either AAV-LUC or AAV-WT-OPT used in neonates (1.7e13 VP/kg) was administered systemically via the femoral vein.

Six weeks after treatment, mice injected with AAV-WT-OPT showed a significantly increased LVEF compared to AAV-LUC treated mice (**Figure 13A**). No significant difference in QRS amplitude or duration between the groups were observed (**Figure 13B****,** **8C**). To uncover any subtle changes in electrical conduction, it was administered a supratherapeutic dose of flecainide (40 mg/kg), a class I antiarrhythmic drug that can induce arrhythmias at high doses (Cerrone M et al. Sodium current deficit and arrhythmogenesis in a murine model of plakophilin-2 haploinsufficiency. Cardiovasc Res 95, 460 (2012)). As shown in **Figure 13D****,** AAV-WT-OPT treatment reduced the AV blocks developed in response to flecainide compared to treatment with AAV-LUC. These findings further support the potential of WT-TMEM43 overexpression therapy in improving cardiac function and electrical conduction in ARVC5, even when administered in adult symptomatic mice.

As summary of these experiments, it has been proved that double transgenic mice overexpressing both WT and mutant TMEM43 forms showed delayed ARVC5 onset, improved cardiac contraction and reduced ECG abnormalities, compared to mice expressing S358L-TMEM43. In addition, cardiomyocyte death and myocardial fibrosis were reduced, with an overall increase in survival. Finally, it has been demonstrated that a single systemic administration of an AAV carrying codon-optimized WT-TMEM43 prevents ventricular dysfunction and ECG abnormalities induced by S358L-TMEM43.

AAV-mediated delivery of WT-TMEM43 offers a promising and specific therapy for patients suffering from this highly lethal disease and a translational tool that may serve as an alternative to current palliative treatments.

### Example 7. Efficacy comparison between the optimized TMEM43 and other TMEM43 variants in the treatment of symptomatic ARVC5 mice.

To compare the efficacy of the codon-optimized TMEM43-WT-OPT with other optimized sequences, the inventors repeated the experiments done for the treatment of symptomatic ARVC5 mice with an AAV bearing another human WT-TMEM43 codon-optimized sequence (TMEM43-WT-UF, WO2023178338). TMEM43-WT-OPT shares 82.88% sequence identity with TMEM43-WT-UF, and each share approximately 78% identity with the native human WT-TMEM43 (**Figure 14A**).

Similar to the AAV-WT-OPT (**Figure 7C**), a self-complementary myotropic AAV that expresses TMEM43-WT-UF under cardiac troponin T promoter was generated (hereafter referred to as AAV-WT-UF).

When delivered systemically to 10-week-old symptomatic mice (1.7e13 VP/kg), AAV-WT-UF treatment resulted in a reduced LVEF compared to AAV-WT-OPT (**Figure 14B**). In addition, mice treated with AAV-WT-UF exhibited an increased tendency to develop fleicainide-induced AV blocks compared to those treated with AAV-WT-OPT (**Figure 14C**). These findings underscore the superior therapeutic efficacy of an AVV carrying TMEM43-WT-OPT over TMEM43-WT-UF for treating ARVC5 in mice.

### SEQUENCE LISTING FREE TEXT

SEQ ID NO: 1: wild-type sequence of TMEM43 
ATGGCCGCGAATTATTCCAGTACCAGTACCCGGAGAGAACATGTCAAAGTTAAAACCA
GCTCCCAGCCAGGCTTCCTGGAACGGCTGAGCGAGACCTCGGGTGGGATGTTTGTG
GGGCTCATGGCCTTCCTGCTCTCCTTCTACCTAATTTTCACCAATGAGGGCCGCGCAT
TGAAGACGGCAACCTCATTGGCTGAGGGGCTCTCGCTTGTGGTGTCTCCCGACAGCA
TCCACAGTGTGGCTCCGGAGAATGAAGGAAGGCTGGTGCACATCATTGGCGCCTTAC
GGACATCCAAGCTTTTGTCTGATCCAAACTATGGGGTCCATCTTCCGGCTGTGAAACT
GCGGAGGCACGTGGAGATGTACCAATGGGTAGAAACTGAGGAGTCCAGGGAGTACAC
CGAGGATGGGCAGGTGAAGAAGGAGACGAGGTATTCCTACAACACTGAATGGAGGTC
AGAAATCATCAACAGCAAAAACTTCGACCGAGAGATTGGCCACAAAAACCCCAGTGCC
ATGGCAGTGGAGTCATTCATGGCAACAGCCCCCTTTGTCCAAATTGGCAGGTTTTTCC
TCTCGTCAGGCCTCATCGACAAAGTCGACAACTTCAAGTCCCTGAGCCTATCCAAGCT
GGAGGACCCTCATGTGGACATCATTCGCCGTGGAGACTTTTTCTACCACAGCGAAAAT
CCCAAGTATCCAGAGGTGGGAGACTTGCGTGTCTCCTTTTCCTATGCTGGACTGAGCG
GCGATGACCCTGACCTGGGCCCAGCTCACGTGGTCACTGTGATTGCCCGGCAGCGG
GGTGACCAGCTAGTCCCATTCTCCACCAAGTCTGGGGATACCTTACTGCTCCTGCACC
ACGGGGACTTCTCAGCAGAGGAGGTGTTTCATAGAGAACTAAGGAGCAACTCCATGAA
GACCTGGGGCCTGCGGGCAGCTGGCTGGATGGCCATGTTCATGGGCCTCAACCTTAT
GACACGGATCCTCTACACCTTGGTGGACTGGTTTCCTGTTTTCCGAGACCTGGTCAAC
ATTGGCCTGAAAGCCTTTGCCTTCTGTGTGGCCACCTCGCTGACCCTGCTGACCGTG
GCGGCTGGCTGGCTCTTCTACCGACCCCTGTGGGCCCTCCTCATTGCCGGCCTGGCC
CTTGTGCCCATCCTTGTTGCTCGGACACGGGTGCCAGCCAAAAAGTTGGAGTGA
SEQ ID NO: 2: wild-type sequence of TMEM43 
MAANYSSTSTRREHVKVKTSSQPGFLERLSETSGGMFVGLMAFLLSFYLIFTNEGRALKTA
TSLAEGLSLVVSPDSIHSVAPENEGRLVHIIGALRTSKLLSDPNYGVHLPAVKLRRHVEMYQ
WVETEEESREYTEDGQVKKETRYSYNTEWRSEIINSKNFDREIGHKNPSAMAVESFMATAP
FVQIGRFFLSSGLIDKVDNFKSLSLSKLEDPHVDIIRRGDFFYHSENPKYPEVGDLRVSFSY
AGLSGDDPDLGPAHVVTVIARQRGDQLVPFSTKSGDTLLLLHHGDFSAEEVFHRELRSNS
MKTWGLRAAGWMAMFMGLNLMTRILYTLVDWFPVFRDLVNIGLKAFAFCVATSLTLLTVA
AGWLFYRPLWALLIAGLALVPILVARTRVPAKKLE
SEQ ID NO: 3: mutant TMEM43 S358L 
ATGGCCGCGAATTATTCCAGTACCAGTACCCGGAGAGAACATGTCAAAGTTAAAACCA
GCTCCCAGCCAGGCTTCCTGGAACGGCTGAGCGAGACCTCGGGTGGGATGTTTGTG
GGGCTCATGGCCTTCCTGCTCTCCTTCTACCTAATTTTCACCAATGAGGGCCGCGCAT
TGAAGACGGCAACCTCATTGGCTGAGGGGCTCTCGCTTGTGGTGTCTCCCGACAGCA
TCCACAGTGTGGCTCCGGAGAATGAAGGAAGGCTGGTGCACATCATTGGCGCCTTAC
GGACATCCAAGCTTTTGTCTGATCCAAACTATGGGGTCCATCTTCCGGCTGTGAAACT
GCGGAGGCACGTGGAGATGTACCAATGGGTAGAAACTGAGGAGTCCAGGGAGTACAC
CGAGGATGGGCAGGTGAAGAAGGAGACGAGGTATTCCTACAACACTGAATGGAGGTC
AGAAATCATCAACAGCAAAAACTTCGACCGAGAGATTGGCCACAAAAACCCCAGTGCC
ATGGCAGTGGAGTCATTCATGGCAACAGCCCCCTTTGTCCAAATTGGCAGGTTTTTCC
TCTCGTCAGGCCTCATCGACAAAGTCGACAACTTCAAGTCCCTGAGCCTATCCAAGCT
GGAGGACCCTCATGTGGACATCATTCGCCGTGGAGACTTTTTCTACCACAGCGAAAAT
CCCAAGTATCCAGAGGTGGGAGACTTGCGTGTCTCCTTTTCCTATGCTGGACTGAGCG
GCGATGACCCTGACCTGGGCCCAGCTCACGTGGTCACTGTGATTGCCCGGCAGCGG
GGTGACCAGCTAGTCCCATTCTCCACCAAGTCTGGGGATACCTTACTGCTCCTGCACC
ACGGGGACTTCTCAGCAGAGGAGGTGTTTCATAGAGAACTAAGGAGCAACTCCATGAA
GACCTGGGGCCTGCGGGCAGCTGGCTGGATGGCCATGTTCATGGGCCTCAACCTTAT
GACACGGATCCTCTACACCTTGGTGGACTGGTTTCCTGTTTTCCGAGACCTGGTCAAC
ATTGGCCTGAAAGCCTTTGCCTTCTGTGTGGCCACCTTGCTGACCCTGCTGACCGTGG
CGGCTGGCTGGCTCTTCTACCGACCCCTGTGGGCCCTCCTCATTGCCGGCCTGGCCC
TTGTGCCCATCCTTGTTGCTCGGACACGGGTGCCAGCCAAAAAGTTGGAGTGA
SEQ ID NO: 4: mutant TMEM43 S358L 
MAANYSSTSTRREHVKVKTSSQPGFLERLSETSGGMFVGLMAFLLSFYLIFTNEGRALKTA
TSLAEGLSLVVSPDSIHSVAPENEGRLVHIIGALRTSKLLSDPNYGVHLPAVKLRRHVEMYQ
WVETEEESREYTEDGQVKKETRYSYNTEWRSEIINSKNFDREIGHKNPSAMAVESFMATAP
FVQIGRFFLSSGLIDKVDNFKSLSLSKLEDPHVDIIRRGDFFYHSENPKYPEVGDLRVSFSY
AGLSGDDPDLGPAHVVTVIARQRGDQLVPFSTKSGDTLLLLHHGDFSAEEVFHRELRSNS
 
MKTWGLRAAGWMAMFMGLNLMTRILYTLVDWFPVFRDLVNIGLKAFAFCVATLLLTLLLTVA
AGWLFYRPLWALLIAGLALVPILVARTRVPAKKL
SEQ ID NO: 5: codon optimized TMEM43 
ATGGCCGCCAACTACTCCTCTACCTCCACCAGGAGAGAGCACGTCAAGGTGAAGACA
TCCTCCCAGCCCGGCTTCCTGGAGAGGCTGAGTGAAACCTCTGGCGGCATGTTTGTG
GGCCTGATGGCCTTCCTGCTGTCTTTTTACCTGATCTTTACAAACGAGGGCAGGGCCC
TGAAGACAGCCACCTCTCTGGCTGAAGGCCTTAGCCTGGTCGTGTCCCCAGATTCTAT
CCACAGCGTGGCTCCTGAGAACGAGGGAAGATTGGTGCACATTATCGGGGCCCTGCG
CACCTCCAAACTGCTGAGTGACCCCAACTACGGCGTGCACCTCCCCGCCGTGAAACT
GAGACGCCATGTGGAGATGTACCAGTGGGTCGAGACCGAGGAAAGCCGAGAGTATAC
CGAGGATGGCCAGGTGAAGAAGGAGACTCGCTACAGCTACAACACCGAGTGGAGATC
CGAGATCATCAACTCCAAGAACTTCGACAGGGAGATCGGACATAAGAACCCATCAGCC
ATGGCCGTGGAGTCTTTCATGGCCACTGCTCCTTTCGTGCAGATCGGCCGGTTCTTCC
TGAGCTCTGGCCTGATCGACAAGGTGGACAACTTCAAGAGCCTGTCCCTGTCTAAGCT
GGAGGATCCTCACGTGGATATCATCCGCAGGGGAGATTTCTTCTACCACAGCGAGAA
CCCAAAATATCCTGAGGTGGGAGACCTGAGAGTGAGCTTCTCCTACGCCGGCCTGAG
CGGCGATGACCCCGATCTGGGCCCCGCCCACGTGGTGACAGTGATCGCCCGACAGC
GCGGCGATCAGCTGGTGCCATTCAGCACCAAGAGCGGTGACACCCTGCTGCTGCTGC
ACCATGGGGATTTTTCCGCTGAGGAGGTGTTCCACAGGGAGCTGCGGAGCAATAGTA
TGAAGACATGGGGGCTGAGGGCCGCAGGCTGGATGGCCATGTTCATGGGACTGAAC
CTGATGACAAGGATCCTGTACACACTGGTGGACTGGTTTCCTGTGTTCAGAGACCTGG
TGAATATTGGCCTGAAGGCCTTCGCTTTCTGCGTGGCCACCAGCCTGACCCTGCTGAC
CGTGGCCGCCGGGTGGCTGTTCTACAGGCCACTCTGGGCTCTGCTGATCGCCGGGC
TGGCCCTGGTCCCTATCCTCGTGGCTAGAACTAGAGTGCCAGCTAAGAAGCTGGAAT
GA
SEQ ID NO: 6 codon optimized TMEM43-UF 
ATGGCCGCAAACTACTCAAGCACCTCTACACGACGGGAGCACGTCAAAGTGAAAACCT
CATCTCAGCCTGGATTCCTGGAGCGACTGAGCGAGACATCCGGCGGCATGTTCGTGG
GCCTGATGGCCTTCCTGCTGAGCTTTTACCTGATCTTCACCAACGAGGGCAGGGCCCT
GAAGACAGCCACATCTCTGGCCGAGGGCCTGAGCCTGGTGGTGTCTCCCGACAGCAT
CCACTCTGTGGCACCTGAGAATGAGGGCAGGCTGGTGCACATCATCGGCGCCCTGCG
CACATCCAAGCTGCTGTCTGATCCTAATTATGGAGTGCACCTGCCAGCAGTGAAGCTG
CGGAGACACGTGGAGATGTACCAGTGGGTGGAGACAGAGGAGAGCCGCGAGTATAC
CGAGGACGGCCAGGTGAAGAAGGAGACACGGTACTCCTATAACACCGAGTGGAGAAG
CGAGATCATCAACTCCAAGAATTTTGATAGGGAGATCGGCCACAAGAATCCATCCGCC
 
ATGGCCGTGGAGTCTTTTATGGCAACAGCACCCTTCGTGCAGATTGGCCGCTTCTTTC
TGAGCTCCGGCCTGATCGACAAGGTGGATAACTTCAAGTCTCTGAGCCTGTCCAAGCT
GGAGGACCCCCACGTGGATATCATCAGGCGCGGCGACTTCTTTTACCACTCTGAGAAT
CCCAAGTATCCTGAAGTGGGCGATCTGCGGGTGTCTTTTAGCTATGCAGGCCTGTCTG
GCGACGATCCAGACCTGGGACCTGCACACGTGGTGACAGTGATCGCAAGACAGAGAG
GCGATCAGCTGGTGCCTTTCTCTACAAAGTCTGGCGACACCCTGCTGCTGCTGCACCA
CGGCGACTTCAGCGCCGAGGAGGTGTTCCACAGGGAGCTGCGCTCCAACTCTATGAA
GACATGGGGCCTGAGAGCAGCAGGATGGATGGCCATGTTTATGGGCCTGAATCTGAT
GACACGGATCCTGTACACCCTGGTGGACTGGTTCCCCGTGTTCCGGGACCTGGTGAA
TATCGGCCTGAAGGCCTTCGCCTTTTGCGTGGCCACCTCCCTGACACTGCTGACAGT
GGCAGCAGGATGGCTGTTCTATAGACCACTGTGGGCCCTGCTGATCGCCGGCCTGGC
TCTGGTCCCAATCCTGGTGGCAAGAACTCGGGTCCCTGCTAAGAAACTGGAATAA
SEQ ID NO: 7 human TMEM43 forward
   TCCCAAGTATCCAGAGGTGGGAGACT
SEQ ID NO: 8 human TMEM43 reverse
   AGGCCGGCAATGAGGAGGG
SEQ ID NO: 9 LOX forward
   GCTGCGGAAGAAAACTGC
SEQ ID NO: 10 LOX reverse
   CCTTGGTTCTTCACTCTTTGC
SEQ ID NO: 11 POSTN forward
   AACGTCTGTGCCCTCCAG
SEQ ID NO: 12 POSTN reverse
   AGCCTTTCATCCCTTCCATT
SEQ ID NO: 13 COL1A1 forward
   GTGCCACTCTGACTGGAAGA
SEQ ID NO: 14 COL1A1 reverse
   CTGACCTGTCTCCATGTTGC
SEQ ID NO: 15 COL3A1 forward
   CACCCTTCTTCATCCCACTC
SEQ ID NO: 16 COL3A1 reverse
   ATGTCATCGCAAAGGACAGA
SEQ ID NO: 17 ACTA1 forward
   GACCACAGCTGAACGTGAGA
SEQ ID NO: 18 ACTA1 reverse
   TGTTGTAGGTGGTCTCATGGAT
SEQ ID NO: 19 Luciferase forward
   TATCATGGCCTCGTGAAATCC
SEQ ID NO: 20 Luciferase reverse
   TCCTGGGTCCGATTCAATAAAC
SEQ ID NO: 21 cardiac troponin T promoter (cTnT) 
GGGATAAAAGCAGTCTGGGCTTTCACATGACAGCATCTGGGGCTGCGGCAGAGGGTC
GGGTCCGAAGCGCTGCCTTATCAGCGTCCCCAGCCCTGGGAGGTGACAGCTGGCTG
GCTTGTGTCAGCCCCTCGGGCACTCACGTATCTCCGTCCGACGGGTTTAAAATAGCAA
AACTCTGAGGCCACACAATAGCTTGGGCTTATATGGGCTCCTGTGGGGGAAGGGGGA
GCACGGAGGGGGCCGGGGCCGCTGCTGCCAAAATAGCAGCTCACAAGTGTTGCATT
CCTCTCTGGGCGCCGGGCACATTCCTGCTGGCTCTGCCCGCCCCGGGGTGGGCGCC
GGGGGGACCTTAAAGCCTCTGCCCCCCAAGGAGCCCTTCCCAGACAGCCGCCGGCA
CCCACCGCTCCGTGGGA
SEQ ID NO: 22 SV40 late polyA signal

CAGACATGATAAGATACATTGATGAGTTTGGACAAACCACAACTAGAATGCAGTGAAAA
AAATGCTTTATTTGTGAAATTTGTGATGCTATTGCTTTATTTGTAACCATTATAAGCTGC
AATAAACAAGTTAACAACAACAATTGCATTCATTTTATGTTTCAGGTTCAGGGGGAGGT
GTGGGAGGTTTTTTAAAGCAAGTAAAACCTCTACAAATGTGGTA

## Claims

1. A nucleic acid comprising the human TMEM43 coding sequence as set forth in SEQ ID NO: 5.

2. The nucleic acid of claim 1, wherein the nucleic acid is a recombinant adeno-associated virus (AAV) vector comprising the sequence as set forth in SEQ ID NO: 5.

3. The nucleic acid of claim 2, wherein the nucleic acid is a single-stranded or self-complementary AAV nucleic acid vector.

4. A pharmaceutical composition comprising a plurality of the nucleic acid of any of claims 1 to 4.

5. The pharmaceutical composition of claim 4 further comprising a pharmaceutically acceptable carrier.

6. The pharmaceutical compositions of claims 4 or 5 for use as a medicament.

7. The pharmaceutical compositions of claims 5 or 6 for use in a method of treating a disease linked to TMEM43 mutations and/or a disease linked to TMEM43 downregulation.

8. The pharmaceutical composition for use according to claim 7, wherein the disease linked to is selected from TMEM43 mutations is selected from Emery-Dreifuss-related myopathy and autosomal dominant auditory neuropathy spectrum disorder or an arrhythmogenic cardiomyopathy, and the disease linked to TMEM43 downregulation is selected from cardiac hypertrophy and sepsis-induced cardiomyopathy.

9. The pharmaceutical composition for use of claim 8, wherein the arrhythmogenic cardiomyopathy is arrhythmogenic right ventricular cardiomyopathy type 5 (ARVC5).

10. A method of increasing the expression of human TMEM43 of SEQ ID NO: 2 in a target cell, comprising contacting a target cell with a plurality of AAV particles comprising a nucleic acid expression cassette comprising the functional human TMEM43 coding sequence as set forth in SEQ ID NO: 1, the cardiac specific promoter cTNT and the SV40 late polyA signal.

11. A nucleic acid expression cassette comprising a functional human TMEM43 coding sequence as set forth in SEQ ID NO: 1 operable linked to the cTnT promoter of SEQ ID NO: 21, for use in a method for treating a cardiac disease.

12. The nucleic acid expression cassette for use according to claim 11, wherein the cardiac disease is selected from cardiac hypertrophy, sepsis-induced cardiomyopathy, or an arrhythmogenic cardiomyopathy.

13. The nucleic acid expression cassette for use according to claim 11, wherein the cardiac disease is arrhythmogenic cardiomyopathy type 5 (ARVC5).
